# EUROPEAN PATENT APPLICATION

(11) **EP 2 251 380 A2**
(43) Date of publication of application: **17.11.2010**
(21) Application number: 10250924.7
(22) Date of filing: 14.05.2010
(51) Int. Cl.: C09B 23/06, C09B 23/08, G01N 33/58

(54) **Dye conjugates and methods of use**

(30) Priority: 15.05.2009 US 178870 P
(71) Applicant: Millipore Corporation, Billerica, MA 01821 (US)
(72) Inventor: Dehghani, Ali, Campbell, CA 95008 (US); Tyagarajan, Kamala, Fremont, CA 94539 (US)
(74) Representative: Setna, Rohan P.

(57) **Abstract**

The present invention relates to cyanine compounds, compositions comprising them, articles of manufacture, and methods of making and using them. The cyanines usefully comprise one or more carboxyl groups or derivatives thereof that are indirectly attached to an aryl ring on an alkylaryl cyanine substituent. Also provided are conjugates of the disclosed cyanines and one or more other substances. Solvates, solutions, and derivatized forms of the cyanines are also provided. The cyanines find use in a variety of bioassays, both in direct single-label applications and in energy transfer formats. Methods utilizing the disclosed cyanines for analyzing a sample for a target are provided. Also disclosed are detection complexes comprising the disclosed cyanines, as well as compositions and articles comprising the cyanines in an excited state, for example formed by direct excitation or by energy transfer. The cyanines may be used as alternatives to other known optically active species in a variety of settings. Other embodiments are described further herein.

## Description

### TECHNICAL FIELD

This invention relates to optically active cyanine compounds, compositions comprising such materials, methods of making and using them, and articles incorporating them.

### BACKGROUND OF THE INVENTION

Cyanine compounds have been used for over a hundred years. Historically, cyanines have been used as sensitizers for photographic emulsions and in some cases for textile dyeing. More recently, cyanines have been used in CD-R and DVD-R media.

Cyanines have recently found particular use as fluorescent labels in bioassays, either as single labels or in energy transfer schemes employing multiple labels. The explosion of bioinformatics, array technology, and genome projects over the last decade has led to a great need for environmentally acceptable labels such as fluorophores to provide information on the physical sequence of biomolecules, on the expression of genes at the polynucleotide and protein level, and on the actual location of biomolecules in cells, tissues and organisms. Fluorescent labels can also be used to detect cell-specific markers and characterize and separate specific cell populations and subpopulations using cytometric methods. These techniques typically use a fluorescent molecule such as a cyanine conjugated to a biomolecule such as an antibody or a nucleotide or dye terminator.

Energy transfer schemes have frequently been used in bioassays, and often employ cyanines. Energy transfer involves excitation of one species by an energy source and transfer of energy from the excited species to a second species, which may then emit detectable energy or transfer it to a subsequent species. Energy transfer schemes have proven particularly useful in fluorescent sequencing, where dye terminators comprising spectrally unique combinations of fluors specific for each of four terminating nucleotides are used to provide single-reaction sequencing information for each residue of a polynucleotide. In some schemes, different fluor combinations may employ initial fluor(s) that can all be excited by the same energy source, but are coupled to other fluors or fluor combinations that provide a unique emission wavelength for each different combination. Such methods can greatly expand the types and amounts of information that can be provided from a single experiment, and can also expand the uses of apparatuses providing a single excitation source, permitting greater amounts of information to be obtained while efficiently using reagents and available sample.

There is a need in the art for optically active cyanines, for methods of making and using them, and for compositions, and articles of manufacture comprising such compounds.

### SUMMARY OF THE INVENTION

The present invention relates to cyanine compounds, compositions and articles of manufacture comprising them, and methods of making and using them. The cyanines usefully comprise one or more carboxyl groups or derivatives thereof that are indirectly attached to an aryl ring on an alkylaryl cyanine substituent. Also provided are conjugates of the disclosed cyanines and one or more other substances. Solvates, solutions, and derivatized forms of the cyanines are also provided. The cyanines find use in a variety of bioassasys, both in direct single-label applications and in energy transfer formats. Methods utilizing the disclosed cyanines for analyzing a sample for a target are provided. Also disclosed are detection complexes comprising the disclosed cyanines, as well as compositions and articles comprising the cyanines in an excited state formed by direct excitation or by energy transfer. The cyanines may be used as alternatives to other known optically active species in a variety of settings. Other embodiments are described further herein.

### BRIEF DESCRIPTION OF THE DRAWINGS

Figure 1 depicts the absorption spectra of cyanines NGy5 and Gy5.
Figure 2 depicts the absorption spectra of PE-NGy5 conjugates formed by reacting different ratios of cyanine to phycoerythrin (PE).
Figure 3 depicts the emission spectra of PE-NGy5 conjugates formed at different coupling ratios of cyanine to protein (532 nm excitation).
Figure 4 depicts the emission spectra of PE-NGy5 and PE-Gy5 cyanine conjugates (532 nm excitation).
Figure 5 depicts the absorption spectra of PE-NGy7 conjugates at different coupling ratios.
Figure 6 depicts the fluorescence emission spectra of PE-NGy7 conjugates at different coupling ratios.
Figure 7 depicts the fluorescence excitation and emission spectra of a Streptavidin-NGy3 Conjugate.
Figure 8 depicts the emission spectra of Streptavidin-Gy3 and Streptavidin-NGy3 conjugates.

### DETAILED DESCRIPTION OF THE INVENTION

The present invention relates to cyanine compounds, compositions comprising them, articles of manufacture, and methods of making and using them. It was discovered that certain cyanines comprising carboxyl-substituted alkylaryl substituents exhibited an unexpected lack of fluorescence emission. The present invention results from experiments in which moving the carboxyl group from direct attachment to the aryl ring was shown to improve fluorescence properties, while retaining the useful carboxyl functionality.

The compounds of the invention may be used in a variety of applications. The compounds are desirably optically active, and exhibit absorption and/or emission properties. The compounds can thus be utilized in applications where absorption of energy of particular wavelengths is desired, in applications where emission of energy is desired, and in applications where both absorption and emission are desired. The compounds may be used as labels for substances including biomolecules by coupling or recruiting the compounds to particular substances and/or locations. The compounds may be used to form conjugates with other substances. The compounds may be used in energy transfer experiments, and may be provided in transfer complexes or in forms which can be recruited to the location of other optically active substances with which they exhibit energy transfer. The compounds can be used as photographic sensitizers, in dye lasers, as saturable absorbers for passively Q-switching lasers, and as molecular probes of membrane potential. The compounds may be used in a variety of applications in which labels of biomolecules are used, including in labeling of primary, secondary (or subsequent) antibodies, in labeling of nucleotides that can be incorporated into labeled polynucleotides, including sequencing reactions (e.g., in labeled nucleotides and dye-terminators), in proximity assays used to determine the proximity of two optically active substances in a variety of settings, in apoptotic assays, in photobleaching recovery experiments, in fluorescence correlation spectroscopy, in microarray experiments, in transcriptomics, and in proteomics. Desirably, the compounds of the invention exhibit good solubility in aqueous media.

The compounds may be provided as isolated compounds, as solvates, as solutions, as conjugates, and in other forms as described. Also provided are compositions and articles comprising cyanines of the invention in an excited state, attained either by direct excitation with an electromagnetic source or by energy transfer from another excited species. These articles include conjugated sensors as well as detection complexes employing excited cyanines.

Embodiments of the invention include articles of manufacture utilizing cyanines of the invention. For example, a plurality of labeled sensors comprising cyanines can be used simultaneously in an array. Multiplex embodiments may employ 2, 3, 4, 5, 10, 15, 20, 25, 50, 100, 200, 400, 1000, 5000, 10000, 50000 or more distinct articles incorporating one or more embodiments described herein. Other aspects of the invention are discussed further herein.

Before the present invention is described in further detail, it is to be understood that this invention is not limited to the particular methodology, articles, compositions or apparatuses described, as such methods, articles, compositions or apparatuses can, of course, vary. It is also to be understood that the terminology used herein is for the purpose of describing particular embodiments only, and is not intended to limit the scope of the present invention.

Use of the singular forms "a," "an," and "the" include plural references unless the context clearly dictates otherwise. Thus, for example, reference to "a cyanine" includes a plurality of cyanines, reference to "a solvent" includes a plurality of such solvents, reference to "an ester" includes a plurality of esters, and the like. Additionally, use of specific plural references, such as "two," "three," etc., read on larger numbers of the same subject unless the context clearly dictates otherwise. The term "or" when used herein as the sole conjunction means "and/or" unless stated otherwise. The term "including" and related terms such as "includes" as used herein are not limiting and allow for the presence of elements in addition to those specifically recited.

Terms such as "connected," "attached," and "linked" are used interchangeably herein and encompass direct as well as indirect connection, attachment, linkage or conjugation unless the context clearly dictates otherwise.

Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and all such ranges are encompassed within the invention. Where a value being discussed has inherent limits, for example where a component can be present at a concentration of from 0 to 100%, or where the pH of an aqueous solution can range from 1 to 14, those inherent limits are specifically disclosed as are ranges based on those inherent limits. Where a value is explicitly recited, it is to be understood that values which are about the same quantity or amount as the recited value are also within the scope of the invention, as are ranges based thereon with any other value as described herein.

Where a combination or group of elements is disclosed, each subset of those elements is also specifically disclosed and is within the scope of the invention. Conversely, where different elements or groups of elements are disclosed, combinations thereof are also disclosed.

Where any element of an invention is disclosed as having a plurality of alternatives, examples of that invention in which each alternative is excluded singly or in any combination with the other alternatives are also hereby disclosed; more than one element of an invention can have such exclusions, and all combinations of elements having such exclusions are hereby disclosed.

Unless defined otherwise or the context clearly dictates otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

All publications mentioned herein are hereby incorporated by reference for the purpose of disclosing and describing the particular materials and methodologies for which the reference was cited. The publications discussed herein are provided solely for their disclosure prior to the filing date of the present application. Nothing herein is to be construed as an admission that the invention is not entitled to antedate such disclosure by virtue of prior invention.

### DEFINITIONS

In describing the present invention, the following terms will be employed, and are intended to be defined as indicated below.

"Activated" or "activating" as used herein, for example in connection with the terms "group," "alkyl group" and "carboxylic acid ester," refers to groups comprising at least one reactive moiety useful for attachment to other molecules (e.g., having available functional groups, for example amino, hydroxy and/or sulfhydryl groups). Exemplary reactive moieties include such groups containing isothiocyanate, isocyanate, monochlorotriazine, dichlorotriazine, mono- or di-halogen substituted pyridine, mono- or di-halo substituted diazine, maleimide, aziridine, sulfonyl halide, acid halide, acid anhydride, hydroxysuccinimide ester, hydroxysulfosuccinimide ester, imido ester, hydrazine, azidonitrophenyl, azide, 3-(2-pyridyl dithio)-proprionamide, glyoxal, aldehyde, and a polymerizable group.

A "coupling pair" refers to two chemical moieties which can react to form a bond. One or both members of a coupling pair may be activating groups. A member of a coupling pair may be a functional group in a species of interest, and may be formed during initial synthesis or introduced subsequently; exemplary functional groups include carboxylic and sulfonic acids, amines, hydroxyls, thiols, aldehydes, cyano, and tyrosine. Exemplary bonds that a coupling pair may form include amide, amine, ester, thiol, thioester, disulfide, carbonyl, ether and polymeric linkages.

"Alkyl" refers to a branched, unbranched or cyclic saturated hydrocarbon group of 1 to 24 carbon atoms optionally substituted at one or more positions, and includes polycyclic compounds. Examples of alkyl groups include optionally substituted methyl, ethyl, n-propyl, isopropyl, n-butyl, s-butyl, t-butyl, n-pentyl, isopentyl, neopentyl, n-hexyl, n-heptyl, n-octyl, n-decyl, hexyloctyl, tetradecyl, hexadecyl, eicosyl, tetracosyl and the like, as well as cycloalkyl groups such as cyclopropyl, cyclobutyl, cyclopentyl, cyclohexyl, cycloheptyl, cyclooctyl, adamantyl, and norbornyl. The term "lower alkyl" refers to an alkyl group of 1 to 6 carbon atoms, preferably 1 to 4 carbon atoms. Exemplary substituents on substituted alkyl groups include hydroxyl, cyano, alkoxy, =O, =S, -NO₂, halogen, haloalkyl, heteroalkyl, carboxyalkyl, amine, amide, thioether and -SH.

"Alkoxy" refers to an "-Oalkyl" group, where alkyl is as defined above. A "lower alkoxy" group intends an alkoxy group containing one to six, more preferably one to four, carbon atoms.

"Alkenyl" refers to an unsaturated branched, unbranched or cyclic hydrocarbon group of 2 to 24 carbon atoms containing at least one carbon-carbon double bond and optionally substituted at one or more positions. Examples of alkenyl groups include ethenyl, 1-propenyl, 2-propenyl (allyl), 1-methylvinyl, cyclopropenyl, 1-butenyl, 2-butenyl, isobutenyl, 1,4-butadienyl, cyclobutenyl, 1-methylbut-2-enyl, 2-methylbut-2-en-4-yl, prenyl, pent-1-enyl, pent-3-enyl, 1,1-dimethylallyl, cyclopentenyl, hex-2-enyl, 1-methyl-1-ethylallyl, cyclohexenyl, heptenyl, cycloheptenyl, octenyl, cyclooctenyl, decenyl, tetradecenyl, hexadecenyl, eicosenyl, tetracosenyl and the like. Preferred alkenyl groups herein contain 2 to 12 carbon atoms. The term "lower alkenyl" intends an alkenyl group of 2 to 6 carbon atoms, preferably 2 to 4 carbon atoms. The term "cycloalkenyl" intends a cyclic alkenyl group of 3 to 8, preferably 5 or 6, carbon atoms. Exemplary substituents on substituted alkenyl groups include hydroxyl, cyano, alkoxy, =O, =S, -NO₂, halogen, haloalkyl, heteroalkyl, amine, thioether and -SH.

"Alkenyloxy" refers to an "-Oalkenyl" group, wherein alkenyl is as defined above.

"Alkylaryl" refers to an alkyl group that is covalently joined to an aryl group. Preferably, the alkyl is a lower alkyl. An alkylaryl group may optionally be substituted on either or both of the alkyl and aryl components with substituents, as described herein. Exemplary alkylaryl groups include benzyl, phenethyl, phenopropyl, 1-benzylethyl, phenobutyl, 2-benzylpropyl, 3-naphthylpropenyl and the like.

"Alkylaryloxy" refers to an "-Oalkylaryl" group, where alkylaryl is as defined above.

"Alkynyl" refers to an unsaturated branched or unbranched hydrocarbon group of 2 to 24 carbon atoms containing at least one -C=C- triple bond, optionally substituted at one or more positions. Examples of alkynyl groups include ethynyl, n-propynyl, isopropynyl, propargyl, but-2-ynyl, 3-methylbut-1-ynyl, octynyl, decynyl and the like. Preferred alkynyl groups herein contain 2 to 12 carbon atoms. The term "lower alkynyl" intends an alkynyl group of 2 to 6, preferably 2 to 4, carbon atoms, and one -C=C- triple bond. Exemplary substituents on substituted alkynyl groups include hydroxyl, cyano, alkoxy, =O, =S, -NO₂, halogen, haloalkyl, heteroalkyl, amine, thioether and -SH.

"Amide" refers to -C(O)NR'R" and to - S(O)₂ NR'R", where R' and R" are independently selected from hydrogen, alkyl, aryl, and alkylaryl.

"Amine" refers to an -N(R')R" group, where R' and R" are independently selected from hydrogen, alkyl, aryl, and alkylaryl.

"Aryl" refers to a group that has at least one aromatic ring having a conjugated pi electron system with delocalized pi electrons satisfying Huckel's rule, and includes carbocyclic, heterocyclic, bridged and/or polycyclic aryl groups, and can be optionally substituted at one or more positions. Typical aryl groups contain 1 to 5 aromatic rings, which may be fused and/or linked. Exemplary aryl groups include phenyl, furanyl, azolyl, thiofuranyl, pyridyl, pyrimidyl, pyrazinyl, triazinyl, biphenyl, indenyl, benzofuranyl, indolyl, naphthyl, quinolinyl, isoquinolinyl, quinazolinyl, pyridopyridinyl, pyrrolopyridinyl, purinyl, tetralinyl and the like. Exemplary substituents on optionally substituted aryl groups include alkyl, alkoxy, alkylcarboxy, alkenyl, alkenyloxy, alkenylcarboxy, aryl, aryloxy, alkylaryl, alkylaryloxy, fused saturated or unsaturated optionally substituted rings, halogen, haloalkyl, heteroalkyl, -S(O)R, sulfonyl, -SO₃R, -SR, -NO₂, -NRR', -OH, -CN, -C(O)R, -OC(O)R, - NHC(O)R, -(CH₂)ₙCO₂R or -(CH₂)ₙCONRR' where n is 0-4, and wherein R and R' are independently H, alkyl, aryl or alkylaryl.

"Aryloxy" refers to an "-Oaryl" group, where aryl is as defined above.

"Carbocyclic" refers to an optionally substituted compound containing at least one ring and wherein all ring atoms are carbon, and can be saturated or unsaturated.

"Carbocyclic aryl" refers to an optionally substituted aryl group wherein the ring atoms are carbon.

"Conjugated" and "conjugated system" refers to molecular entities in which a group or chain of atoms bears valence electrons that are not engaged in single-bond formation and that modify the behaviour of each other. Conjugated polymers are polymers exhibiting such delocalized bonding. Typically conjugated systems can comprise alternating single and double or multiple bonds form conjugated systems, and can be interspersed with atoms (e.g., heteroatoms) comprising nonbonding valence electrons. In some embodiments, conjugated polymers can comprise aromatic repeat units, optionally containing heteroatom linkages.

"Halo" or "halogen" refers to fluoro, chloro, bromo or iodo. "Halide," "fluoride," "chloride" and the like refer to the anionic form of a halogen when used with reference to a noncovalently bound halogen anion; "acid halide" and the like refers to moieties in which a hydroxyl group of a corresponding acid is replaced with a halogen, typically forming an activated species useful for coupling reactions.

"Haloalkyl" refers to an alkyl group substituted at one or more positions with a halogen, and includes alkyl groups substituted with only one type of halogen atom as well as alkyl groups substituted with a mixture of different types of halogen atoms. Exemplary haloalkyl groups include trihalomethyl groups, for example trifluoromethyl.

"Heteroalkyl" refers to an alkyl group wherein one or more carbon atoms and associated hydrogen atom(s) are replaced by an optionally substituted heteroatom, and includes alkyl groups substituted with only one type of heteroatom as well as alkyl groups substituted with a mixture of different types of heteroatoms. Heteroatoms include oxygen, sulfur, and nitrogen. As used herein, nitrogen heteroatoms and sulfur heteroatoms include any oxidized form of nitrogen and sulfur, and any form of nitrogen having four covalent bonds including protonated and alkylated forms. An optionally substituted heteroatom refers to a heteroatom having one or more attached hydrogens optionally replaced with alkyl, aryl, alkylaryl and/or hydroxyl. The term "lower heteroalkyl" refers to a heteroalkyl group of 1 to 6 carbon and heteroatoms, preferably 1 to 4 carbon and heteroatoms.

"Heterocyclic" refers to a compound containing at least one saturated or unsaturated ring having at least one heteroatom and optionally substituted at one or more positions. Typical heterocyclic groups contain 1 to 5 rings, which may be fused and/or linked, where the rings each contain five or six atoms. Examples of heterocyclic groups include piperidinyl, morpholinyl and pyrrolidinyl. Exemplary substituents for optionally substituted heterocyclic groups are as for alkyl and aryl at ring carbons and as for heteroalkyl at heteroatoms.

"Heterocyclic aryl" refers to an aryl group having at least 1 heteroatom in at least one aromatic ring. Exemplary heterocyclic aryl groups include furanyl, thienyl, pyridyl, pyridazinyl, pyrrolyl, N-lower alkyl-pyrrolo, pyrimidyl, pyrazinyl, triazinyl, tetrazinyl, triazolyl, tetrazolyl, imidazolyl, bipyridyl, tripyridyl, tetrapyridyl, phenazinyl, phenanthrolinyl, purinyl and the like.

"Hydrocarbyl" refers to hydrocarbyl substituents containing 1 to about 20 carbon atoms, including branched, unbranched and cyclic species as well as saturated and unsaturated species, for example alkyl groups, alkylidenyl groups, alkenyl groups, alkylaryl groups, aryl groups, and the like. The term "lower hydrocarbyl" intends a hydrocarbyl group of one to six carbon atoms, preferably one to four carbon atoms.

A "substituent" refers to a group that replaces one or more hydrogens attached to a carbon or nitrogen. Exemplary substituents include alkyl, alkylidenyl, alkylcarboxy, alkoxy, alkenyl, alkenylcarboxy, alkenyloxy, aryl, aryloxy, alkylaryl, alkylaryloxy, -OH, amide, carboxamide, carboxy, sulfonyl, =O, =S, -NO₂, halogen, haloalkyl, fused saturated or unsaturated optionally substituted rings, -S(O)R, -SO₃R, -SR, -NRR', -OH, -CN, -C(O)R, - OC(O)R, -NHC(O)R, -(CH₂)ₙCO₂R or -(CH₂)ₙCONRR' where n is 0-4, and wherein R and R' are independently H, alkyl, aryl or alkylaryl. Substituents also include replacement of a carbon atom and one or more associated hydrogen atoms with an optionally substituted heteroatom.

"Sulfonyl" refers to -S(O)₂R, where R is alkyl, aryl, -C(CN)=C-aryl, -CH₂CN, or alkylaryl.

"Thioamide" refers to -C(S)NR'R", where R' and R" are independently selected from hydrogen, alkyl, aryl, and alkylaryl.

"Thioether" refers to -SR, where R is alkyl, aryl, or alkylaryl.

The terms "polynucleotide," "oligonucleotide," "nucleic acid" and "nucleic acid molecule" are used herein to include a polymeric form of nucleotides of any length, and may comprise ribonucleotides, deoxyribonucleotides, analogs thereof, or mixtures thereof. This term refers only to the primary structure of the molecule. Thus, the term includes triple-, double- and single-stranded deoxyribonucleic acid ("DNA"), as well as triple-, double- and single-stranded ribonucleic acid ("RNA"). It also includes modified, for example by alkylation, and/or by capping, and unmodified forms of the polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), including tRNA, rRNA, hRNA, and mRNA, whether spliced or unspliced, any other type of polynucleotide which is an N- or C-glycoside of a purine or pyrimidine base, and other polymers containing nonnucleotidic backbones, for example, polyamide (e.g., peptide nucleic acids (PNAs)) and polymorpholino (commercially available from the Anti-Virals, Inc., Corvallis, Oregon, as Neugene) polymers, and other synthetic sequence-specific nucleic acid polymers providing that the polymers contain nucleobases in a configuration which allows for base pairing and base stacking, such as is found in DNA and RNA. There is no intended distinction in length between the terms "polynucleotide," "oligonucleotide," "nucleic acid" and "nucleic acid molecule," and these terms are used interchangeably herein. These terms refer only to the primary structure of the molecule. Thus, these terms include, for example, 3'-deoxy-2',5'-DNA, oligodeoxyribonucleotide N3' P5' phosphoramidates, 2'-O-alkyl-substituted RNA, double- and single-stranded DNA, as well as double- and single-stranded RNA, and hybrids thereof including for example hybrids between DNA and RNA or between PNAs and DNA or RNA, and also include known types of modifications, for example, labels, alkylation, "caps," substitution of one or more of the nucleotides with an analog, internucleotide modifications such as, for example, those with uncharged linkages (e.g., methyl phosphonates, phosphotriesters, phosphoramidates, carbamates, etc.), with negatively charged linkages (e.g., phosphorothioates, phosphorodithioates, etc.), and with positively charged linkages (e.g., aminoalkylphosphoramidates, aminoalkylphosphotriesters), those containing pendant moieties, such as, for example, proteins (including enzymes (e.g. nucleases), toxins, antibodies, signal peptides, poly-L-lysine, etc.), those with intercalators (e.g., acridine, psoralen, etc.), those containing chelates (of, e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide.

It will be appreciated that, as used herein, the terms "nucleoside" and "nucleotide" will include those moieties which contain not only the known purine and pyrimidine bases, but also other heterocyclic bases which have been modified. Such modifications include methylated purines or pyrimidines, acylated purines or pyrimidines, or other heterocycles. Modified nucleosides or nucleotides can also include modifications on the sugar moiety, e.g., wherein one or more of the hydroxyl groups are replaced with halogen, aliphatic groups, or are functionalized as ethers, amines, or the like. The term "nucleotidic unit" is intended to encompass nucleosides and nucleotides.

Furthermore, modifications to nucleotidic units include rearranging, appending, substituting for or otherwise altering functional groups on the purine or pyrimidine base which form hydrogen bonds to a respective complementary pyrimidine or purine. The resultant modified nucleotidic unit optionally may form a base pair with other such modified nucleotidic units but not with A, T, C, G or U. Abasic sites may be incorporated which do not prevent the function of the polynucleotide. Some or all of the residues in the polynucleotide can optionally be modified in one or more ways.

Standard A-T and G-C base pairs form under conditions which allow the formation of hydrogen bonds between the N3-H and C4-oxy of thymidine and the Nl and C6-NH2, respectively, of adenosine and between the C2-oxy, N3 and C4-NH2, of cytidine and the C2-NH2, N'-H and C6-oxy, respectively, of guanosine. Thus, for example, guanosine (2-amino-6-oxy-9-β-D-ribofuranosyl-purine) may be modified to form isoguanosine (2-oxy-6-amino-9-P-D-ribofuranosyl-purine). Such modification results in a nucleoside base which will no longer effectively form a standard base pair with cytosine. However, modification of cytosine (1-β-D-ribofuranosyl-2-oxy-4-amino-pyrimidine) to form isocytosine (1-β-D-ribofuranosyl-2-amino-4-oxy-pyrimidine) results in a modified nucleotide which will not effectively base pair with guanosine but will form a base pair with isoguanosine. Isocytosine is available from Sigma Chemical Co. (St. Louis, MO); isocytidine may be prepared by the method described by Switzer et al. (1993) Biochemistry 32:10489-10496 and references cited therein; 2'-deoxy-5-methyl-isocytidine may be prepared by the method of Tor et al. (1993) J. Am. Chem. Soc. 115:4461-4467 and references cited therein; and isoguanine nucleotides may be prepared using the method described by Switzer et al. (1993), supra, and Mantsch et al. (1993) Biochem. 14:5593-5601, or by the method described in U.S. Patent No. 5,780,610 to Collins et al. Other nonnatural base pairs may be synthesized by the method described in Piccirilli et al. (1990) Nature 343:33-37 for the synthesis of 2,6-diaminopyrimidine and its complement (1-methylpyrazolo-[4,3]pyrimidine-5,7-(4H,6H)-dione. Other such modified nucleotidic units which form unique base pairs are known, such as those described in Leach et al. (1992) J. Am. Chem. Soc. 114:3675-3683 and Switzer et al., supra.

"Nucleic acid probe" and "probe" are used interchangeably and refer to a structure comprising a polynucleotide, as defined above, that contains a nucleic acid sequence that can bind to a corresponding analyte. The polynucleotide regions of probes may be composed of DNA, and/or RNA, and/or synthetic nucleotide analogs.

"Complementary" or "substantially complementary" refers to the hybridization or base pairing between nucleotides or nucleic acids, such as, for instance, between the two strands of a double stranded DNA molecule or between a polynucleotide primer and a primer binding site on a single stranded nucleic acid to be sequenced or amplified. Complementary nucleotides are, generally, A and T (or A and U), or C and G. Two single stranded RNA or DNA molecules are said to be substantially complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%.

Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa Nucleic Acids Res. 12:203 (1984).

Stringent hybridization conditions will typically include salt concentrations of less than about 1M, more usually less than about 500 mM and preferably less than about 200 mM. Hybridization temperatures can be as low as 5° C, but are typically greater than 22° C, more typically greater than about 30° C, and preferably in excess of about 37° C. Longer fragments may require higher hybridization temperatures for specific hybridization. Other factors may affect the stringency of hybridization, including base composition and length of the complementary strands, presence of organic solvents and extent of base mismatching, and the combination of parameters used is more important than the absolute measure of any one alone.

"Aptamer" (or "nucleic acid antibody") is used herein to refer to a single- or doublestranded polynucleotide that recognizes and binds to a molecule of interest by virtue of its shape. See, e.g., PCT Publication Nos. WO 92/14843, WO 91/19813, and WO 92/05285.

"Polypeptide" and "protein" are used interchangeably herein and include a molecular chain of amino acids linked through peptide bonds. The terms do not refer to a specific length of the product. Thus, "peptides," "oligopeptides," and "proteins" are included within the definition of polypeptide. The terms include polypeptides containing [post-translational] modifications of the polypeptide, for example, glycosylations, acetylations, phosphorylations, and sulphations. In addition, protein fragments, analogs (including amino acids not encoded by the genetic code, e.g. homocysteine, ornithine, D-amino acids, and creatine), natural or artificial mutants or variants or combinations thereof, fusion proteins, derivatized residues (e.g. alkylation of amine groups, acetylations or esterifications of carboxyl groups) and the like are included within the meaning of polypeptide. By "modified" with reference to proteins (including antibodies), and other biomolecules, is meant a modification in one or more functional groups, for example any portion of an amino acid, the structure and/or location of a sugar or other carbohydrate, or other substituents of biomolecules, and can include without limitation chemical modifications (e.g., succinylation, acylation, the structure and/or location of disulfide bonds), as well as noncovalent binding (e.g., of a small molecule, including a drug).

"Amino acid" includes both natural amino acid and substituted amino acids. "Natural amino acid" refers to any of the commonly occurring amino acids as generally accepted in the peptide art and represent L-amino acids unless otherwise designated (with the exception of achiral amino acids such as glycine), including the canonical 20 amino acids encoded directly by the genetic code, as well as selenocysteine, selenomethionine, and ornithine. "Substituted amino acid" refers to an amino acid containing one or more additional chemical moieties that are not normally a part of the amino acid. Such substitutions can be introduced by a targeted deriviatizing agent that is capable of reacting with selected side chains or terminal residues and via other art-accepted methods. For example, cysteinyl residues most commonly are reacted with alpha-haloacetates (and corresponding amines), such as chloroacetic acid or chloroacetamide, to give carboxymethyl or carboxyamidomethyl derivatives. Cysteinyl residues can also be derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole. Carboxyl side groups (aspartyl or glutamyl) can be selectively modified by reaction with carbodiimides such as 1-cyclohexyl-3-(2-morpholinyl-(4-ethyl) carbodiimide or 1-ethyl-3 (4 azonia 4,4-dimethylpentyl) carbodiimide. Aspartyl and glutamyl residues can be converted to asparaginyl and glutaminyl residues by reaction with ammonium ions. Glutaminyl and asparaginyl residues can be deamidated to the corresponding glutamyl and aspartyl residues. Alternatively, these residues can be deamidated under mildly acidic conditions. Other modifications include hydroxylation of proline and lysine, phosphorylation of hydroxyl groups of seryl or theonyl residues, methylation of the alpha-amino groups of lysine, arginine and histidine side chains (see, e.g., T. E. Creighton, Proteins: Structure and Molecule Properties, W. H. Freeman & Co., San Francisco, pp. 79-86 (1983)), acetylation of the N-terminal amine, and amidation of C-terminal carboxyl groups. Blocking groups and/or activating groups can also be incorporated.

As used herein, the term "binding pair" refers to first and second molecules that bind specifically to each other with greater affinity than to other components in the sample. The binding between the members of the binding pair is typically noncovalent. Exemplary binding pairs include immunological binding pairs (e.g. any haptenic or antigenic compound in combination with a corresponding antibody or binding portion or fragment thereof, for example digoxigenin and anti-digoxigenin, fluorescein and anti-fluorescein, dinitrophenol and anti-dinitrophenol, bromodeoxyuridine and anti-bromodeoxyuridine, mouse immunoglobulin and goat anti-mouse immunoglobulin), IgG and protein A, IgG and protein G, IgG and protein L, and nonimmunological binding pairs (e.g., biotin and a biotin binding substance [including avidin, streptavidin, or a derivative of either thereof], nucleotides and nucleotide-binding proteins, hormone [e.g., thyroxine and cortisol]-hormone binding protein, receptor-receptor agonist or antagonist (e.g., acetylcholine receptor-acetylcholine or an analog thereof) IgG-protein A, lectin-carbohydrate, enzyme-enzyme cofactor, enzyme-enzyme-inhibitor, an organic or inorganic molecule and a biomolecule that binds to the molecule, and two polynucleotides capable of forming nucleic acid duplexes and/or higher order structures) and the like. One or both members of the binding pair can be conjugated to additional molecules.

The term "antibody" as used herein includes antibodies obtained from both polyclonal and monoclonal preparations, as well as: hybrid (chimeric) antibody molecules (see, for example, Winter et al. (1991) Nature 349:293-299; and U.S. Patent No. 4,816,567); F(ab')2 and F(ab) fragments; Fv molecules (noncovalent heterodimers, see, for example, Inbar et al. (1972) Proc Natl Acad Sci USA 69:2659-2662; and Ehrlich et al. (1980) Biochem 19:4091-4096); single-chain Fv molecules (sFv) (see, for example, Huston et al. (1988) Proc Natl Acad Sci USA 85:5879-5883); dimeric and trimeric antibody fragment constructs; minibodies (see, e.g., Pack et al. (1992) Biochem 31:1579-1584; Cumber et al. (1992) J Immunology 149B:120-126); humanized antibody molecules (see, for example, Riechmann et al. (1988) Nature 332:323-327; Verhoeyan et al. (1988) Science 239:1534-1536; and U.K. Patent Publication No. GB 2,276,169, published 21 September 1994); and, any functional fragments obtained from such molecules, wherein such fragments retain specific-binding properties of the parent antibody molecule.

As used herein, the term "monoclonal antibody" refers to an antibody composition having a homogeneous antibody population. The term is not limited regarding the species or source of the antibody, nor is it intended to be limited by the manner in which it is made. Thus, the term encompasses antibodies obtained from murine hybridomas, as well as human monoclonal antibodies obtained using human hybridomas or from murine hybridomas made from mice expression human immunoglobulin chain genes or portions thereof. See, e.g., Cote, et al. Monoclonal Antibodies and Cancer Therapy, Alan R. Liss, 1985, p. 77.

"Multiplexing" herein refers to an assay or other analytical method in which multiple analytes can be assayed simultaneously.

"Optional" or "optionally" means that the subsequently described event or circumstance may or may not occur, and that the description includes instances where said event or circumstance occurs singly or multiply and instances where it does not occur at all. For example, the phrase "optionally substituted alkyl" means an alkyl moiety that may or may not be substituted and the description includes both unsubstituted, monosubstituted, and polysubstituted alkyls.

### OPTICALLY ACTIVE CYANINES

Optically active cyanines are provided having the general formula 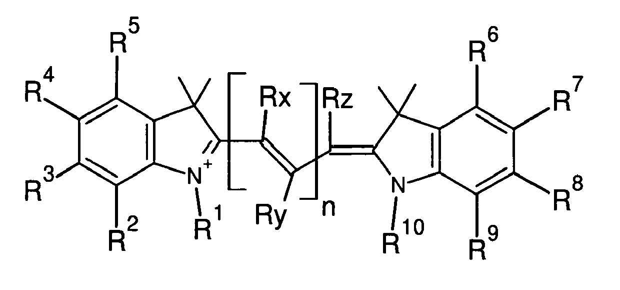 where:
n is zero, 1, 2 or 3;
R¹ and R¹⁰ are independently selected from the group consisting of optionally substituted alkyl, optionally substituted heteroalkyl, and optionally substituted alkylaryl, wherein at least one of R¹ and R¹⁰ is substituted alkylaryl comprising on the aryl component a substituted alkyl or heteroalkyl substituent comprising a carboxylic acid substituent;
R² to R⁹ are independently selected from the group consisting of H, -SO₃H, optionally substituted alkyl, or optionally substituted heteroalkyl, wherein any two adjacent members of R² to R⁹ taken together can form an optionally substituted 5-7 membered mono- or poly-unsaturated fused ring optionally containing one or more ring heteroatoms;
wherein any of R¹ to R¹⁰ can be additionally optionally substituted with a member of a coupling pair;
Rz and each repeat of Rx and Ry are independently selected from the group consisting of H, alkyl, alkoxy, heteroalkyl, heteroalkyloxy, -CN, or wherein any two adjacent members of Rx, Ry, and Rz may be covalently joined to form an optionally substituted 4-7 membered mono- or poly-unsaturated ring optionally containing one or more ring heteroatoms;
or an isomer, ester, amide, acid halide, acid anhydride, and/or salt thereof;
or a mixture of any thereof.

The molecules exhibit optical activity, showing absorption and/or emission of electromagnetic energy. The molecules may desirably fluoresce, and may participate in energy exchange reactions in a variety of formats.

Compounds of the invention may desirably exhibit useful properties for a variety of applications, including good solubility in aqueous or predominantly aqueous media, good purification characteristics, ease of conjugation to other substances, and good solubility and purification properties of conjugates thus produced.

It is understood that, although particular isomers of the compounds may be shown, all isomers of the described compounds, and mixtures of such isomers, are within the scope of the invention.

Of particular interest are those embodiments where one or both of R¹ and R¹⁰ are substituted alkylaryl comprising on the aryl component a substituted alkyl or heteroalkyl substituent comprising a carboxylic acid substituent or a derivative thereof. It was found previously that p-carboxy substitution on an alkylaryl substituent at positions corresponding to R¹ and R¹⁰ could lead to a decrease in cyanine emission. As carboxyl groups impart desirable solubility and coupling properties to these compounds, the working embodiments were synthesized to move the carboxyl group from direct attachment to the aryl ring and thereby disrupt any effect resulting from conjugation of the carbonyl moiety with the aryl ring. Moving the carboxyl group from direct attachment to the aryl ring was found to impart fluorescence emission to a corresponding structure that lacked fluorescence when the carboxyl group was directly bound to the aryl group.

Thus, also of interest are those embodiments where the substituted alkyl or heteroalkyl substituent to which the carboxylic acid substituent is attached is selected from substituted lower alkyl and lower heteroalkyl. Of particular interest are C1-2 alkyl.

Also of interest are embodiments where one or both of the substituted alkylaryl groups at R¹ and/or R¹⁰ are benzyl, phenethyl, or 3-naphthylpropenyl.

The substituted alkylaryl groups are optionally substituted at other positions on their alkyl and aryl components. Other substituents of interest on the substituted aryl moiety of such groups include from 1-4 additional groups selected from =O, =S, acyl, acyloxy, alkyl, alkenyl, alkynyl, heteroalkyl, optionally substituted alkoxy, optionally substituted amino, optionally substituted aryl, optionally substituted aryloxy, azido, carboxylic acid, (optionally substituted alkoxy)carbonyl, (optionally substituted amino)carbonyl, cyano, halogen, optionally substituted heteroaryl, optionally substituted heteroaryloxy, optionally substituted heterocyclyl, optionally substituted heterocyclooxy, hydroxyl, nitro, sulfanyl, sulfinyl, sulfonyl, sulfonic acid, and a member of a coupling pair. Of particular interest are those aryl substituents selected from alkyl, heteroalkyl, alkoxy, amino alkyl, halo, trihalomethyl, and a member of a coupling pair.

Also of interest are those embodiments where at least one or at least two of R² to R⁹ is SO₃H, or a derivative thereof. Particular embodiments of interest include those where one or both of R⁴ and R⁷ are -SO₃H or a derivative thereof. Exemplary derivatives include esters, amides, and acid halides, as well as salts.

Also of interest are those embodiments where n is 1, 2 or 3.

Also of interest are the combinations and subcombinations of the above embodiments of interest, and derivatives thereof, and mixtures thereof. One such combination of embodiments of interest is that wherein:
R¹ and R¹⁰ are each substituted benzyl or substituted phenethyl comprising on the aryl component a substituted lower alkyl or lower heteroalkyl substituent comprising a carboxylic acid substituent;
R⁴ and R⁷ are each sulfonic acid;
   or an isomer, ester, amide, acid halide, acid anhydride, and/or salt thereof;
   or a mixture of any thereof.

Also of interest are the specific working embodiments described herein and referred to by the common names nGy3, nGy5, and nGy7, and derivatives thereof, including isomers, esters, amides, acid halides, acid anhydrides and/or salts thereof, and mixtures thereof particularly for any of these cyanines. Specific compounds of interest include: 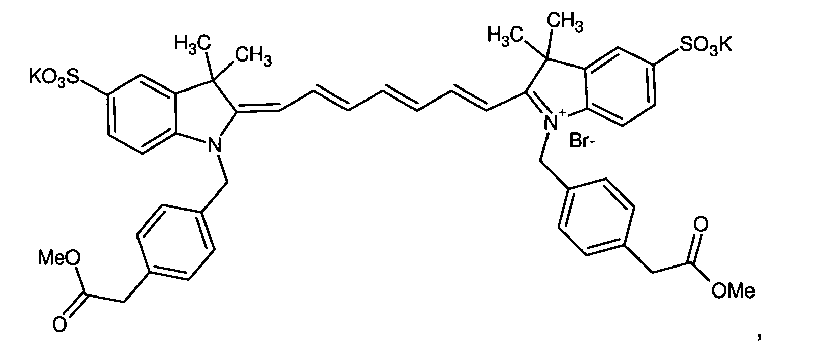 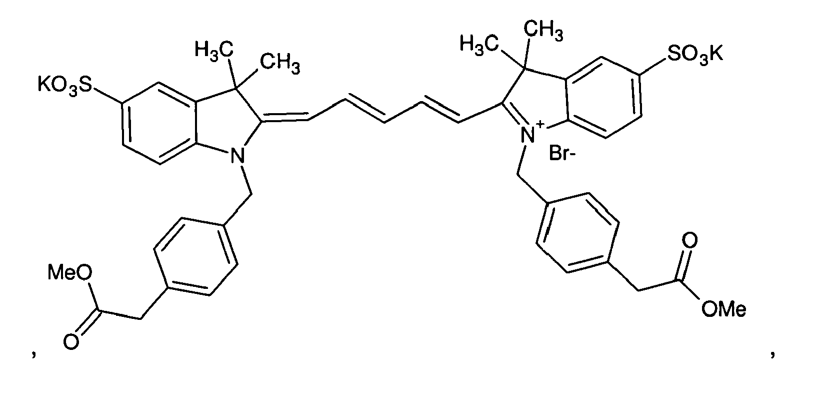 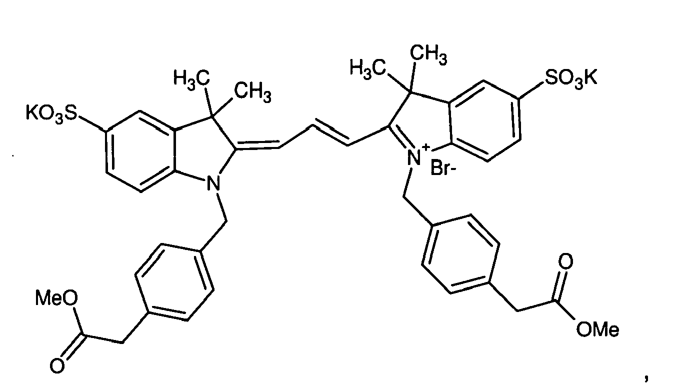 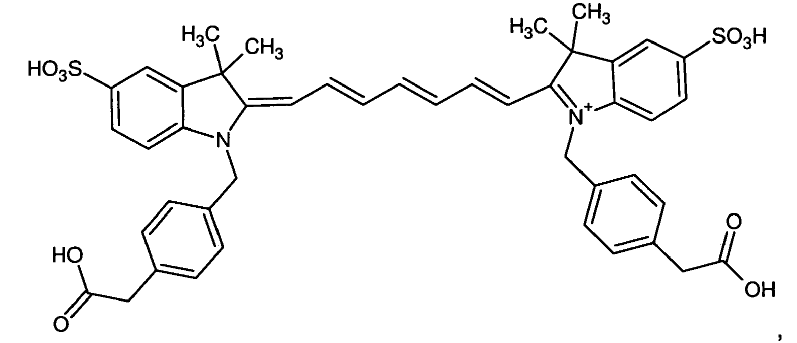 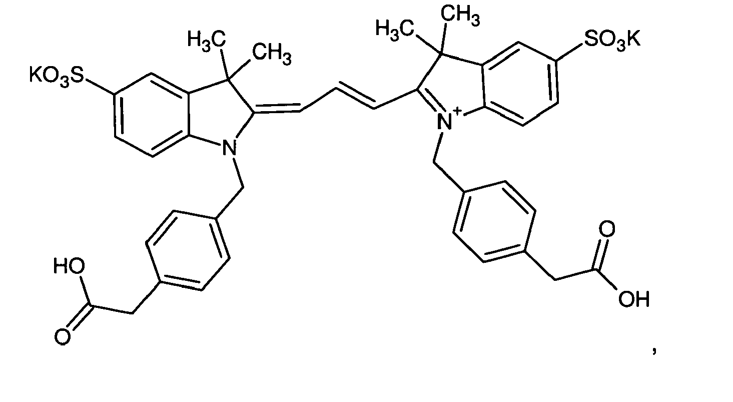 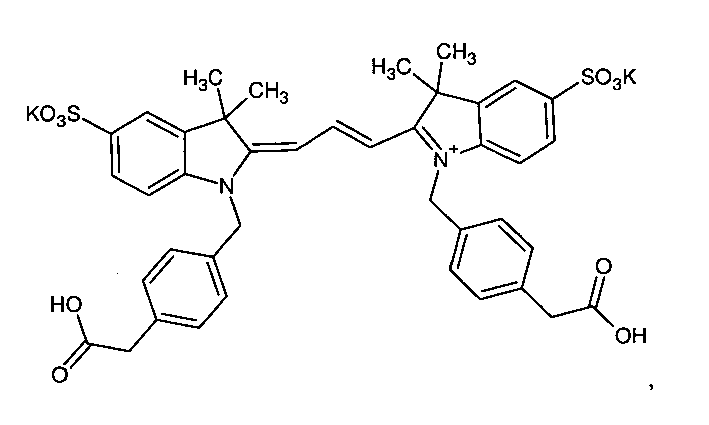 and esters, amides, acid halides, acid anhydrides, isomers, and/or salts of any thereof.

Salts of the described compounds can be prepared through techniques known in the art. By "exchanging," "replacing," "substituting" and the like with relation to the counterions associated with a cyanine is meant exchanging at least 80% of the associated counterions at the desired position. Preferably at least 85% of the counterions are exchanged, more preferably at least 90%, and most preferably 95% or more of the counterions are exchanged. In some cases there may be no detectable levels of the original counterions associated with the compound. Counterion association can be determined by any suitable technique, for example by XPS spectroscopy and/or mass spectrometry. The counterions may be exchanged by any appropriate method known or discoverable in the art. Exemplary ion exchange methods include mass action, dialysis, chromatography, and electrophoresis. After counterion exchange, the new salt form(s) of the cyanine can optionally be purified and/or isolated. Any suitable method(s) that leads towards the purification and/or isolation of the salt of interest can be used. Exemplary methods include crystallization, chromatography (e.g., exclusion, HPLC, FPLC), precipitation, and extraction. Similarly, esters, amides and acid halides can be formed from the described compounds, and purified and/or characterized if desired, using known techniques.

Also of interest are the solvates and solutions produced by dissolving the compounds of the invention in a solvent or solvent mixture. In some embodiments, the cyanines described herein are soluble in aqueous solutions and other highly polar solvents, and can be soluble in water. By "water-soluble" is meant that the material exhibits solubility in a predominantly aqueous solution, which, although comprising more than 50% by volume of water, does not exclude other substances from that solution, including without limitation buffers, blocking agents, cosolvents, salts, metal ions and detergents. Additional solvents which may be used to form solutions, either alone or in combination, include DMSO, DMF, and lower alcohols. Solutions may be provided in a container of any suitable form. Solutions may be packaged in a container designed for incorporation into a solution processing apparatus, for example a printer. In some embodiments, the solution may be provided in an inkjet cartridge designed to be used with a printing device.

### CONJUGATES

Also provided are conjugates of the disclosed cyanines formed by coupling one or more cyanines of the invention to one or more other substances. Exemplary conjugates of interest include those comprising a cyanine of the invention and a biomolecule, a substrate, a probe, a linker, a target, a low affinity false target, a small molecule, one member of a binding pair, a polymer and/or an optically active species (particularly one with which the cyanine may exchange energy), or a combination of any thereof.

Probes and targets form members of binding pairs as described. Targets can include cells, cell fragments, and cell surface molecules, for example immune system molecules, receptors, and markers indicative of specific cell populations or subpopulations. Biomolecules include any species or mixture of species that can be produced by or obtained from a living organism, including cell or bacterial cultures; exemplary biomolecules include proteins, peptides, polynucleotides, polysaccharides, antibodies, triglycerides, lipoproteins, and lectins.

One or more probes may be employed that bind to particular species of targets. The probe and the target may form a binding pair that specifically binds to each other. A sensor biomolecule can be used as a probe that can bind to a target biomolecule. A sensor polynucleotide can be branched, multimeric or circular, but is typically linear, and can contain nonnatural bases. The sensor may be a peptide nucleic acid, the molecular structures of which are well known.

Any polymer can be used to form a conjugate either as a discrete entity or by incorporation into another material of interest, including incorporation on or into a substrate. Exemplary polymers of interest include hydrocarbon polymers (e.g., formed from optionally substituted alkenes and/or optionally substituted alkynes), hydrophilic polymers, heteroalkyl polymers, including polyalkylene oxides including polytheylene oxide and polypropylene oxide, polyamines, and dendrimers. Polymers may be or may incorporate other optically active species.

In some instances, the polymer and/or biomolecule can serve as a carrier for a cyanine of the invention, and may prolong its halflife when used in a physiological setting. For example, a cyanine may be coupled to a serum albumin (e.g., bovine, rabbit, mouse, human), a globulin (e.g., alpha, beta, gamma or immunoglobulins), or a hydrophilic polymer (e.g., a polyalkylene oxide such as polyethylene glycol, polypropylene glycol, or a copolymer thereof).

Optically active species of interest include those with which the cyanine can exchange energy, either directly or through intermediate species. Optically active species can include synthetic dyes, semiconductor nanocrystals, lanthanide chelates, polymers proteins and/or optically active fragments thereof. Exemplary proteins include green fluorescent protein, alternatively colored derivatives thereof, Renilla luciferase, phycoerythrin (PE), phycoerythrin B, phycoerythrin R, B or Y, phycocyanin, and allophycocyanin (APC), and derivatives of any thereof. Some particular conjugates of interest include PE-APC-NGy7, PE-NGy5, and NGy3-APC. Conjugates may include more than one additional optically active species.

Conjugates can be formed by reaction of moieties on their components, and may include linking groups useful for coupling and/or spacing the components appropriately. The components which are used as precursors for the conjugates include or are derivatized to include one or more members of coupling pairs that can react with corresponding members of coupling pairs on other conjugate components. Appropriate blocking strategies can be used as needed to protect functional groups that are not to be used in conjugate formation, as known in the art.

Exemplary coupling schemes include amine coupling, thiol coupling, aldehyde coupling, tyrosine coupling, polymeric coupling, and bifunctional (or polyfunctional) crosslinking agents. Amine coupling can be accomplished through reaction of an amine group on one component with an activated ester on another component (for example an N-hydroxysuccinimide ester). Thiol coupling can be accomplished by reaction of an activated thiol group (e.g., a 2-pyridinyldithio moiety) on one component with a thiol group on another component. Alternatively, a thiol group on one component can be reacted with a maleimide or iodoacetyl group on another component. Aldehyde coupling can be accomplished by reaction of an aldehyde group on one component with a hydrazide group on another component. Tyrosine groups can be coupled to diazo groups. Polymeric coupling can be accomplished by preparing a derivatized monomer or repeat unit linked to a component of interest and then performing a polymerization reaction that incorporates that derivatized monomer. The coupling members may be natively present on the species to be coupled, or may be introduced; chemical schemes for introducing such groups are known. For example, aldehyde groups can be introduced by oxidation of cis-diols (as found in many polyols including sugars, polysaccharides and glycoconjugates) with sodium metaperiodate. Bifunctional and heterobifunctional crosslinking agents can also be used to link functional groups that cannot be otherwise directly; for example, glutaraldehyde may be used to crosslink two amine groups, and maleimide hydrazide can be used to link thiol and formyl groups (Heindel et al., Bioconj. Chem. 2(6):427-30, 1997, Nov.-Dec.).

"Linking groups," or "linkers," can be conjugated to the cyanines of the invention, and can be used to conjugate any of the species of the conjugate to each other. The particular composition of the linking group is not critical. Exemplary linking groups include alkyls, heteroalkyls (e.g., polyethers, alkylamines, polyamines), aryls, heteroaryls, alkylaryls, synthetic polymers, naturally occurring polymers, amino acids, a carbohydrates, polypeptides, or combinations thereof, each optionally substituted as described herein with regard to the components of the linking group. In some embodiments, a linking group may be symmetric, rigid and/or sterically hindered, or may comprise a region having one or more of these properties. The linker may be designed to impose a separation distance suitable for energy transfer between two species to which it is coupled, for example imparting a separation from about 10 to about 100 angstroms. The linker may impose a distance of less than about 100 angstroms, less than about 70 angstroms, less than about 30 angstroms, or less than about 20 angstroms. The linking group may comprise one or more different members of coupling pairs, and typically contains at least two members of a coupling pair to allow for linking of at least two substances.

In some embodiments, the cyanine may be deposited on, coupled or otherwise linked to a substrate. The substrate can comprise a wide range of material, either biological, nonbiological, organic, inorganic, or a combination of any of these. In some embodiments, the substrate can be transparent. The substrate can be a rigid material, for example a rigid plastic or a rigid inorganic oxide. The substrate can be a flexible material, for example a transparent organic polymer such as polyethyleneterephthalate or a flexible polycarbonate. The substrate can be conductive or nonconductive.

The cyanines can be deposited on a substrate in any of a variety of formats. For example, the substrate may be a polymerized Langmuir Blodgett film, functionalized glass, Si, Ge, GaAs, indium doped GaN, GaP, SiC (Nature 430:1009, 2004), SiO2, SiN4, semiconductor nanocrystals, modified silicon, or any of a wide variety of gels or polymers such as (poly)tetrafluoroethylene, (poly)vinylidenedifluoride, polystyrene, cross-linked polystyrene, polyacrylic, polylactic acid, polyglycolic acid, poly(lactide coglycolide), polyanhydrides, poly(methyl methacrylate), poly(ethylene-co-vinyl acetate), polyethyleneterephthalate, polysiloxanes, polymeric silica, latexes, dextran polymers, epoxies, polycarbonates, agarose, poly(acrylamide) or combinations thereof. Conducting polymers and photoconductive materials can be used. The substrate can take the form of a photodiode, an optoelectronic sensor such as an optoelectronic semiconductor chip or optoelectronic thin-film semiconductor, or a biochip.

In some embodiments, the substrate may be particles that are non-uniform/irregular in shape. The particles may have at least two different (X-, Y- and/or Z-) dimensions, and may have three (or more, for unusually shaped particles) different dimensions. The particles are therefore nonspherical, having a shape other than that of a solid sphere. In some embodiments, the particles exhibit an increased surface area over a sphere or other solid shape occupying the same volume. Desirably, the non-uniform particles exhibit an irregular surface (on a macro- and/or micro-scale) that produces a large increase in surface area. The particles desirably exhibit at least a two-fold increase in surface area, and may exhibit at least a three-fold, five-fold, 10-fold or 20-fold increase in surface area. The particles may exhibit up to a 30-fold, 40-fold, 50-fold, 100-fold, or 200-fold increase in surface area over a similarly sized smooth spherical particle. The particles may exhibit an increased binding capacity over a similarly-sized spherical particle, which may result from the increased surface area and/or from an increase in the density of capture moieties (or derivatizable functionalities) used to bind analyte. Desirably, at least one, two or three (or all) dimensions of the particle may be less than about 30 or 40 microns, as is compatible with flow cytometric systems, and may be less than about 20 microns, less than about 10 microns, or less than about 2 microns in such dimensions. With reference to these dimensions, it is understood that such particles are typically provided as distributions of different sizes, and that particles will exhibit mean distributions meeting this limitation, such that an average particle in a population will meet such limitation(s). The particles may be generally bead-like, although lacking a uniform spherical surface, and may be porous, microporous or macroporous, or may be nonporous. Particles having a mean diameter of less than 2 microns may be desirable, as they can exhibit improved suspension properties which can lead to increased contact with the test sample and/or higher binding capacities.

Conjugates can comprise more than one additional substance in addition to the cyanine. For example, a conjugate may comprise a cyanine, an optically active species with which the cyanine can exchange energy, and a probe or sensor for a target of interest. Such a conjugate may also include a low affinity false target, which blocks the probe/sensor component in the absence of the target of interest and binds at a lower affinity than the target, and thereby can reduce or eliminate background signals formed from spurious binding of the probe/sensor region in the absence of target.

Particular cyanine conjugates of interest include those comprising a compound having the formula 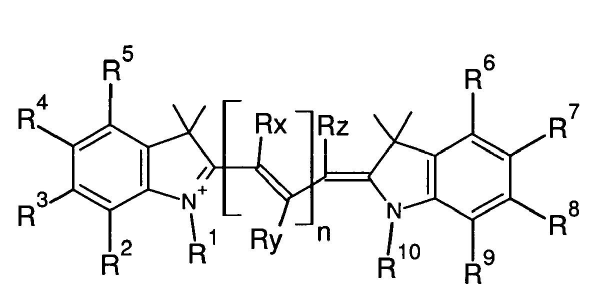 where:
n is zero, 1, 2 or 3;
1 10
R and R are independently selected from the group consisting of optionally substituted alkyl, optionally substituted heteroalkyl, and optionally substituted alkylaryl, wherein at least one of R¹ and R¹⁰ is substituted alkylaryl comprising on the aryl component a substituted alkyl or heteroalkyl substituent comprising a carboxylic acid substituent;
R² to R⁹ are independently selected from the group consisting of H, -SO₃H, optionally substituted alkyl, or optionally substituted heteroalkyl, wherein any two adjacent members of R² to R⁹ taken together can form an optionally substituted 5-7 membered mono- or poly-unsaturated fused ring optionally containing one or more ring heteroatoms;
Rz and each repeat of Rx and Ry are independently selected from the group consisting of H, alkyl, alkoxy, heteroalkyl, heteroalkyloxy, -CN, or wherein any two adjacent members of Rx, Ry, and Rz may be covalently joined to form an optionally substituted 4-7 membered mono- or poly-unsaturated ring optionally containing one or more ring heteroatoms;
or an isomer, ester, amide, acid halide, acid anhydride, and/or salt thereof;
   wherein the compound is bound through a member of a coupling pair at any of R¹ to R¹⁰ to a second species selected from a probe, a linker, a target, a polymer, a biomolecule, a low affinity false target, one member of a binding pair, an optically active species and/or a substrate.

Conjugates of interest include those in which the cyanine is conjugated to a substrate, including a bead. The cyanine may be used alone or in combination to label any conjugate of interest, and may be incorporated into coding schemes involving optical labels and/or energy transfer. In some embodiments, the cyanine is conjugated to an irregularly shaped particle.

Also of interest are those conjugates involving a cyanine of the structured described above, wherein:
R¹ and R¹⁰ are each substituted benzyl or substituted phenethyl comprising on the aryl component a substituted lower alkyl or lower heteroalkyl substituent comprising a carboxylic acid substituent;
R⁴ and R⁷ are each sulfonic acid;
or an isomer, ester, amide, acid halide, acid anhydride, acid anhydride, and/or salt thereof;
or a mixture of any thereof.

Also of interest are those conjugates wherein the compound has a structure selected from the group consisting of: 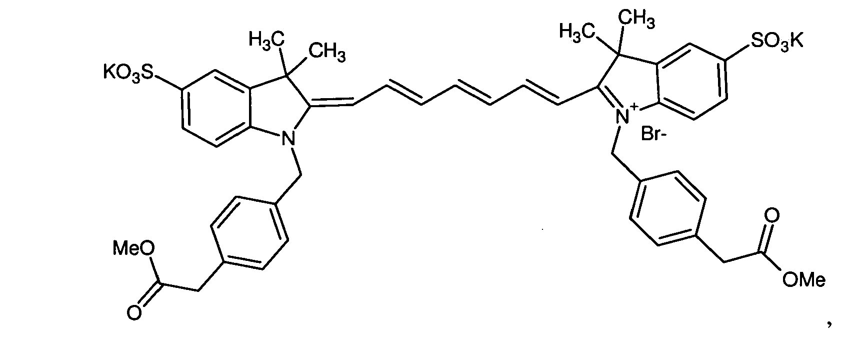 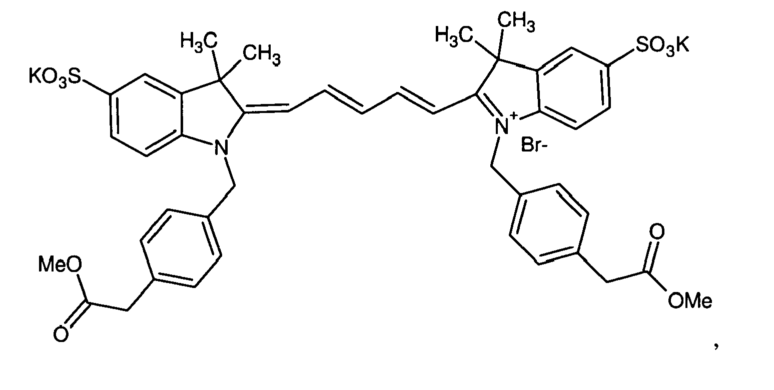 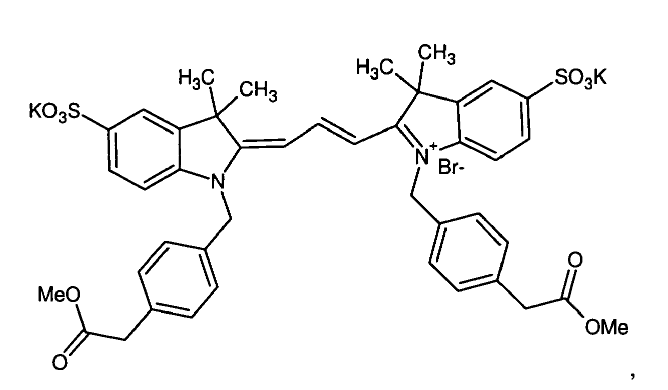 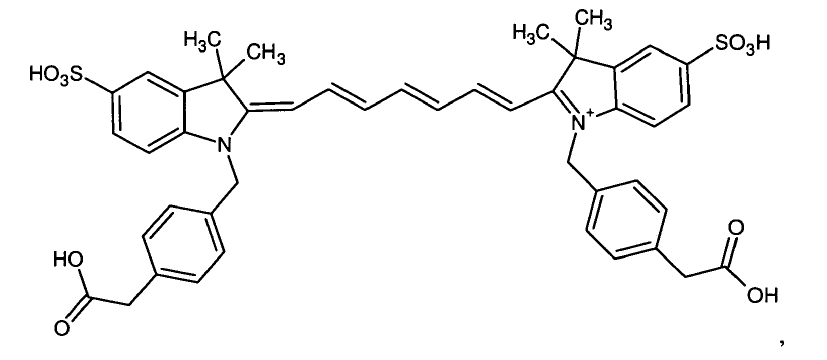 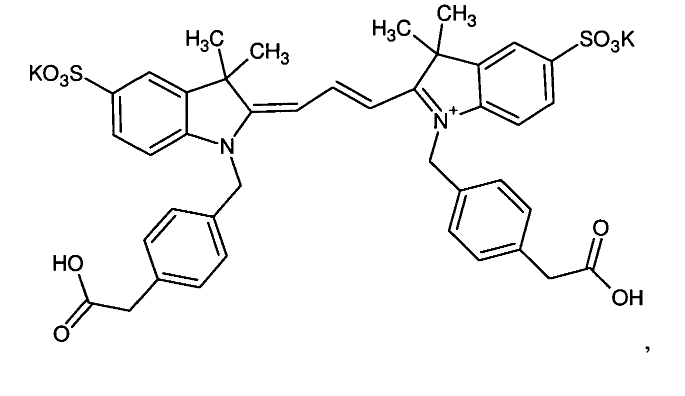 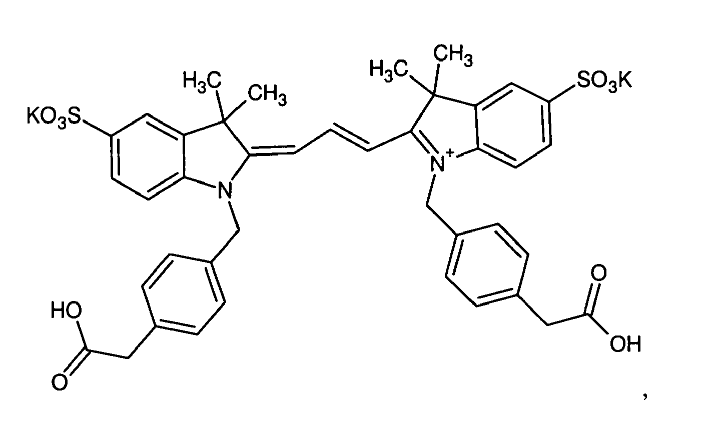 and an ester, amide, acid halide, acid anhydride, isomer, and/or salt of any thereof, wherein the compound as shown is conjugated through at least one of the carboxylic acid groups on the substituted alkylaryl to at least one second species.

### ARTICLES OF MANUFACTURE

The cyanines of the invention can be incorporated into articles of manufacture described herein as well as in articles in which cyanines have previously been used. Exemplary articles of manufacture include conjugates such as derivatized particles or beads, derivatized members of binding pairs, antibody conjugates, derivatized small molecules, biosensors, stains, and can be used in array or microarray form. The cyanines may be used in holographic gratings in combination with synthetic polymers (e.g., vinyl polymers) in information storage devices, for example in CD-R and DVD-R media.

Cyanine labeled species (probes and/or targets) can form detection complexes incorporating the probe, its target, and one or more conjugated cyanines. Also provided are compositions and articles comprising cyanines of the invention in an excited state, attained either by direct excitation with an electromagnetic source or by energy transfer from one or a series of different molecules. These articles include conjugated sensors as well as detection complexes comprising excited cyanines.

Solution processing methods can be used to incorporate cyanines into articles of manufacture where appropriate. Printing techniques may advantageously be used to deposit the cyanines in certain settings, e.g., inkjet printing, offset printing, etc. Where desired, after deposition of a solution comprising a cyanine, the solvent can be removed. Any available method or combination of methods may be used for removing the solvent. Exemplary solvent removal methods include evaporation, heating, extraction, and subjecting the solution to a vacuum, and combinations comprising any thereof.

### METHODS OF USE

The cyanines described herein can be used in a variety of methods, as known for other cyanines and other fluorescent compounds. The cyanines may be used for direct labeling, detection, and/or quantitation of a substance of interest. The cyanines can be used in binding assays, including competitive binding assays, by labeling one member of a binding pair with a cyanine. The cyanines can be bound to a substrate directly or through one or more intermediate species. Conjugated species including conjugated particles can be used for the detection and/or quantitation of a target analyte.

Exemplary methods of use include cytometric settings, sequencing of polynucleotides using for example singly or multiply-labeled nucleotides and/or dye terminators, microarray and nanoarray labeling, coding schemes, and energy transfer experiments. The cyanines may be used in bead-based assays and/or cellular assays. Other biological applications in which cyanines can be used include comparative genomic hybridization, transcriptomics, and proteomics, and as markers in microscopic applications. The cyanines of the invention can serve as donors, acceptors, or both, including in multiple energy transfer schemes in which cyanine(s) of the invention form one or more components.

The cyanines may be used in methods which screen for a property of interest. For example, the materials may be tested for increased fluorescent efficiency, for absorbance wavelength, emission wavelength, conductive properties, and other properties described herein. Cyanines can be used to increase the sensitivity range of photographic emulsions.

In some embodiments, methods of analyzing a sample for a target are provided, comprising providing a sample suspected of containing a target, contacting the sample with a conjugate comprising a cyanine and a probe or sensor under conditions in which the probe can bind to the target, if present, to form a detection complex, contacting the sample or a fraction thereof suspected of comprising the detection complex with an energy source that can be absorbed by or transferred to the compound, and determining if energy has been absorbed or transferred to the complex. Such assays may also include low affinity false targets in the conjugate and/or in the detection complex that can be displaced from the probe region by binding of the conjugate to the actual target.

The target analyte in such assays may be a biomolecule, for example a peptide or protein, a polynucleotide such as DNA or RNA, an antibody, saccharides, oligosaccharides, polysaccharides, etc. Alternatively, the target analyte may be a small molecule, and may be organic or inorganic.

In some embodiments, the sample or portion of the sample comprising or suspected of comprising the analyte can be any source of biological material, including cells, tissue or fluid, including bodily fluids, and the deposits left by that organism, including viruses, mycoplasma, and fossils. Typically, the sample is obtained as or dispersed in a predominantly aqueous medium. Nonlimiting examples of the sample include blood, urine, semen, milk, sputum, mucus, a buccal swab, a lavage, a vaginal swab, a rectal swab, an aspirate, a needle biopsy, a section of tissue obtained for example by surgery or autopsy, plasma, serum, spinal fluid, cerebrospinal fluid, amniotic fluid, lymph fluid, the external secretions of the skin, respiratory, intestinal, and genitourinary tracts, tears, saliva, tumors, organs, samples of in vitro cell culture constituents (including but not limited to conditioned medium resulting from the growth of cells in cell culture medium, putatively virally infected cells, recombinant cells, and cell components, including without limitation hybridoma supernatants producing human or murine antibodies and supernatants from cells producing fragments or modified forms of antibodies or other immunological or secreted proteins), a cellular lysate, and a recombinant library comprising polynucleotide sequences.

The sample can be a positive control sample which is known to contain the analyte. A negative control sample can also be used which, although not expected to contain the analyte is suspected of containing it, and is tested in order to confirm the lack of contamination by the target analyte of the reagents used in a given assay, as well as to determine whether a given set of assay conditions produces false positives (a positive signal even in the absence of analyte in the sample).

The sample can be diluted, dissolved, suspended, purified, extracted or otherwise treated to solubilize or resuspend any target analyte present or to render it accessible to reagents.

### EXCITATION AND DETECTION

Any instrument that provides a wavelength that can excite the cyanine and/or a species with which the cyanine can exchange energy and is shorter than the emission wavelength(s) to be detected can be used for excitation. Commercially available devices can provide suitable excitation wavelengths as well as suitable detection components. Any electromagnetic emission wavelength that can be produced and detected can be used.

Exemplary excitation sources include a broadband UV light source such as a deuterium lamp with an appropriate filter, the output of a white light source such as a xenon lamp or a deuterium lamp after passing through a monochromator to extract out the desired wavelength(s), a continuous wave (cw) gas laser, a solid state diode laser, or any of the pulsed lasers. Emitted light can be detected through any suitable device or technique; many suitable approaches are known in the art.

Incident light wavelengths useful for excitation can include 300 nm to 1000 nm wavelength light. Exemplary useful incident light wavelengths include, but are not limited to, wavelengths of at least about 300, 350, 400, 450, 500, 550, 600, 700, 800 or 900 nm, and may be less than about 1000, 900, 800, 700, 600, 550 or 500 nm. Exemplary useful incident light in the region of 450 nm to 500 nm, 500 nm to 550 nm, 550 nm to 600 nm, 600 nm to 700 nm, and 700 nm to 1000 nm. In certain embodiments, the complexes form an excited state upon illumination with incident light including a wavelength of about 488 nm, about 532 nm, about 594 nm and/or about 633 nm. Additionally, useful incident light wavelengths can include, but are not limited to, 488 nm, 532 nm, 594 nm and 633 nm wavelength light.

Any apparatus that can detect an emission produced from a cyanine or a species to which energy has been transferred may be used, including without limitation microscopes, spectrophotometers, flow cytometers, which may be hydrodynamically focused, imaging systems, imaging flow cytometers, and plate-based imaging systems. Nonlimiting examples of systems useful with the present methods include the Guava® EasyCyte™, the Guava® EasyCyte™ Mini, the Guava® PCA™, the Guava® PCA™-96, the Guava® EasyCyte™ Plus, FACS™ Aria, FACS™ Canto, Beckman Coulter Quanta™, Amnis ImageStream™, Molecular Devices ImageXpress™ apparatuses, and similar devices. Other apparatuses, including plate loading, plate washing, plate rocking, and similar devices useful for handling any assay components may be used.

### EXAMPLES

The following examples are set forth so as to provide those of ordinary skill in the art with a complete description of how to make and use the present invention, and are not intended to limit the scope of what is regarded as the invention. Efforts have been made to ensure accuracy with respect to numbers used (e.g., amounts, temperature, etc.) but some experimental error and deviation should be accounted for. Unless otherwise indicated, parts are parts by weight, temperature is degree centigrade and pressure is at or near atmospheric, and all materials are commercially available.

### Experimental

The following examples illustrate certain embodiments of the invention, including synthesis of optically active compounds, derivatives thereof including activated species for coupling, coupled products comprising the optically active compounds, and characterization of the materials produced. In some instances, although the reactions are shown as producing a particular form of the compound, the compound may be protonated at one or more of the acidic positions, as one or more salts, or as mixtures of any thereof.

Example 1. Synthesis of Starting Material

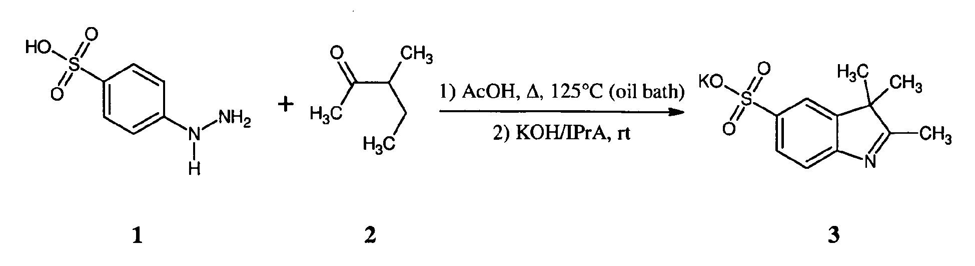

Procedure: A mixture of compounds 1 (10.02g) and 2 (18 mL) in glacial acetic acid is heated to reflux (125° C, oil bath) for 3 hours. During this reaction, the solid went to solution and turned dark red. The reaction mixture is allowed to cool to room temperature to form a pink precipitate. This precipitate is filtered to remove acetic acid, then dried under high vacuum to give 9.90 g of a light pink solid (67% yield). This solid is partially dissolved in methanol (50 mL), then KOH/isopropyl alcohol (40 mL) is added in portions until the mixture becomes basic, providing a yellow precipitate which is collected by filtration and dried under high vacuum. Yield 9.15g, 62.2%.

Example 2. Glutaconaldehyde sodium salt formation from hydrolysis of pyridinium -1-sulfonate.

### [2 - Pentenedial, ION (i-), sodium]

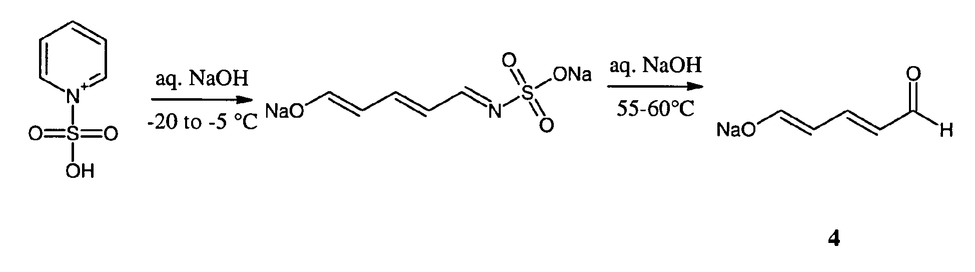

Procedure: A mixture of aq. NaOH (4.3g) is cooled to -20°C and a solution of pyridinium-1-sulfonate (4.8g in 20 mL of water) is added in one portion. The mixture is stirred for 50 minutes at -20°C to -5°C. The mixture is allowed to warm up to room temperature and then is heated to 50-60°C for 1 hour (dark orange). The reaction mixture is allowed to cool to room temperature and then cooled to -10 to -5°C to form a brown precipitate. The brown precipitate is filtered by suction and washed with acetone (3x30 mL). The resulting tan solid is dried under high vacuum at 50°C overnight to give 2.43 g of the desired product. (Org. Syntheses Collective Volume 6, p. 640).

Example 3. Synthesis of Bromomethyl Phenyl Acetic Acid Methyl Ester.

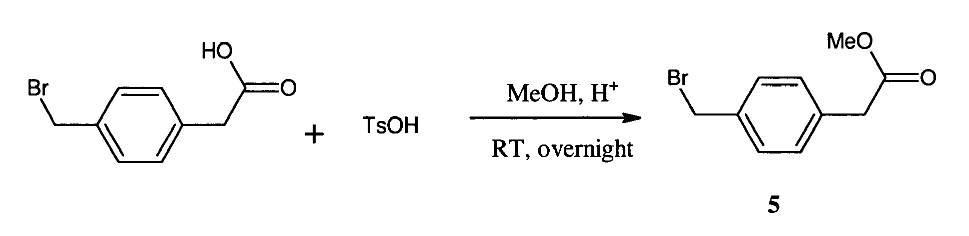

Procedure: A mixture of bromomethyl phenyl acetic acid and TsOH in methanol is stirred at room temperature overnight. Thin layer chromatography (TLC) (20% ethyl acetate) is used to demonstrate the formation of a new spot and disappearance of the starting material. The reaction mixture is concentrated in vacuo to give the crude product 5 as a thick, colorless oil. This crude product is used in the next reaction without further purification.

### Example 4. Synthesis of Substituted Alkaryl Precursor (AD-143-91)

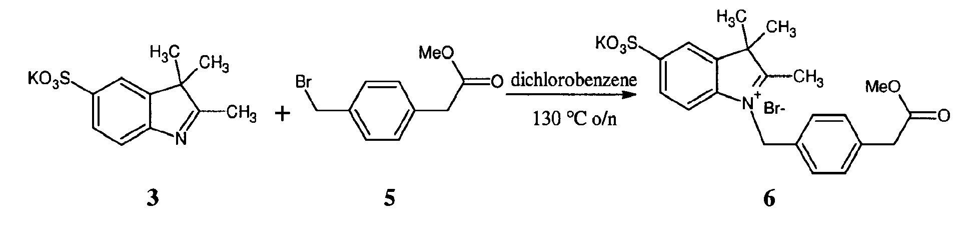

Procedure: A mixture of 1.52g of compound **3** and 2.0g of crude compound **5** in 30 mls of dichlorobenzene is heated to 130°C (oil bath) overnight. A purple solid **6** (AD-143-91) is formed as a precipitate, collected by filtration and dried under high vacuum.

### Example 5. Synthesis of NGy7-OMe.

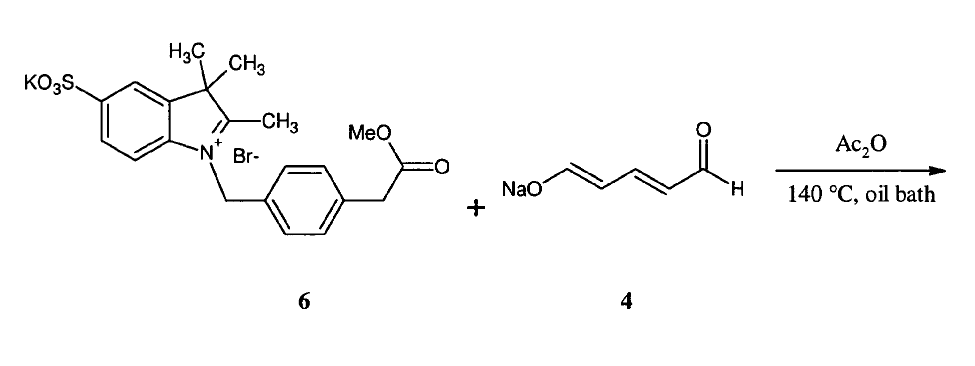

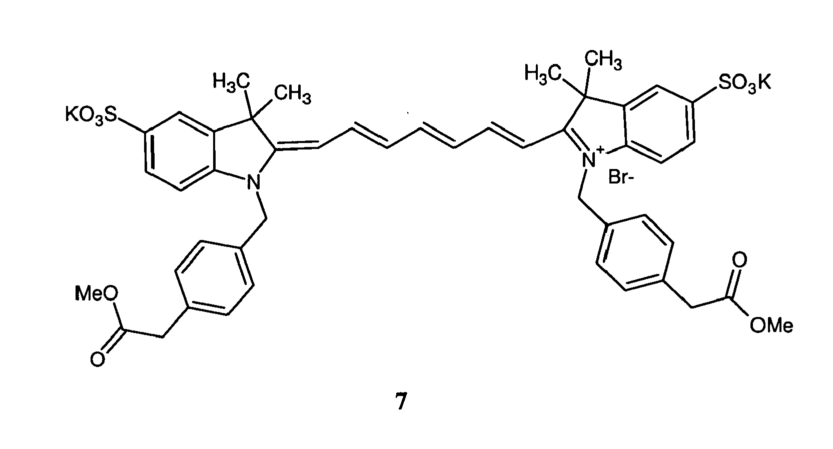

Procedure: To a refluxing solution of 5 mL of acetic anhydride is added a solid mixture of 0.74g of compound 6 and 85 mg of compound **4** at once. The reaction mixture is allowed to cool to room temperature and is stirred for an additional 1.5 hours. 80 mL of ethyl acetate is added and the product is collected by filtration as a dark green solid. Purification by column chromatography (reverse phase H₂0→ 30% acetonitrile in H₂O) furnishes the desired product 7 (NGyOMe).

### Example 6. Synthesis of NGy5-OMe.

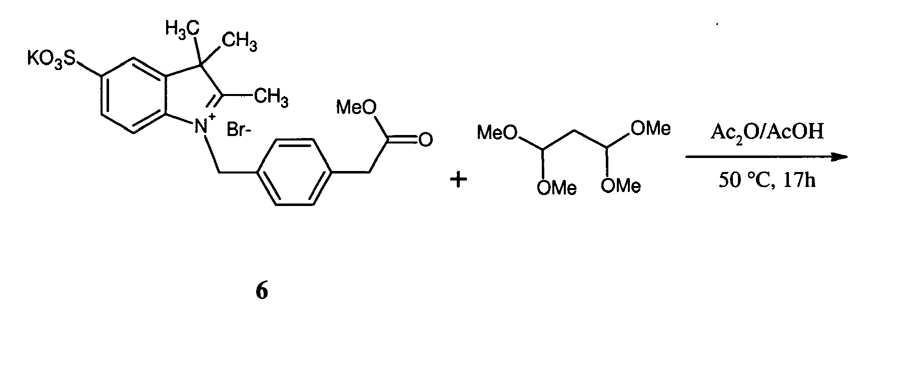

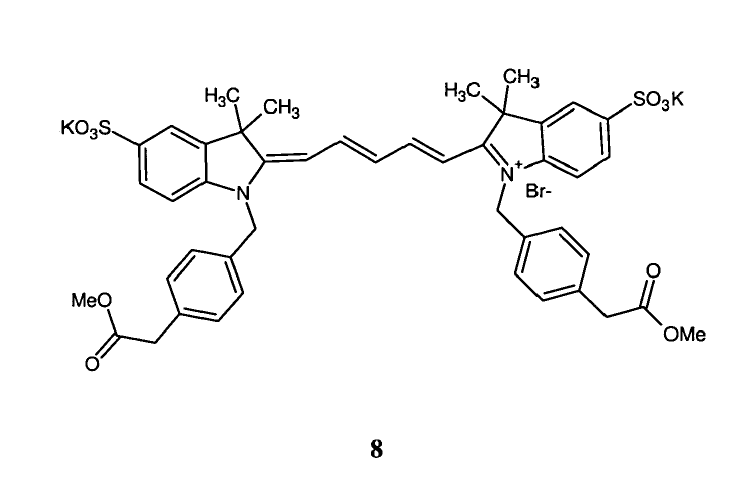

Procedure: A mixture of 521 mg of compound **6**, 566 µl of Ac₂O, 117 µl of AcOH, 2 mL of N-methyl-2-pyrrolidinone, and 165 µl of 1,1,3,3-tetramethyoxypropane is heated to 50°C for 17 hours. TLC (25% AcCN/H₂O) is used to demonstrate formation of the desired product. The mixture is concentrated in vacuo to give crude product **8**. The crude product is purified by prep TLC (1 mm silica gel plate, reverse phase 25% acetonitrile/H₂0).

### Example 7. Synthesis of NGy7-OH.

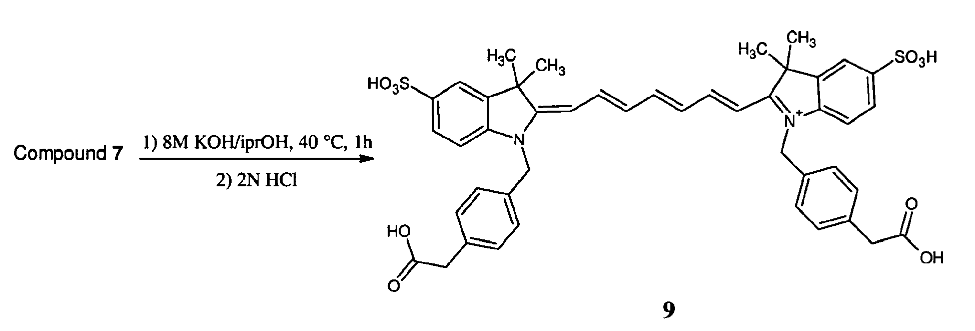

Procedure: A mixture of 44 mg of compound **7** (AD-143-93) and 200 µl of 8M KOH in isopropanol is heated to 40°C for 1 hour. TLC (25% acetonitrile/H₂O) is used to demonstrate completion of the hydrolysis to the desired acid. The mixture is acidified with 850 µl HCI (2N) and dried under vacuum to give the desired product **9** (AD-143-99, NGy7-OH) or a salt or mixture thereof.

### Example 8. Synthesis of NGy7-Bis NHS Ester.

Procedure: A mixture of 80 mg **9** (AD-143-99, NGy7-OH), 90 mg DSC (di(N-succinimidyl) carbodiimide), and 50 ul pyridine in 1 mL of dimethylformamide (DMF) is heated to 55°C (oil bath) overnight to produce the bis-N-hydroxysuccinimide ester of NGy7 (**10**). The reaction can be monitored by TLC (reverse phase, 25% acetonitrile in water); if unreacted starting material remains, 90 mg more DSC and 50 ml more pyridine can be added and heating continued for 5 more hours. The mixture is cooled to room temperature and washed twice with dry ethyl acetate and then with dry dichloromethane. The product **10** is transferred to several vials.

### Example 9. Synthesis of NGy5-OH.

Procedure: A mixture of 227 mg of compound **8** (AD-143-97) (NGy5-OMe) and 0.625 mL KOH/isopropanol in 3 mL water is heated to 40°C for 1 hour. The reaction mixture is neutralized by addition of HCl. Purification of the crude product by reverse phase TLC prep 1 mm plate (25% AcCN/H₂O) furnishes the desired product **11**, NGy5-OH.

### Example 10. Synthesis of NGy5-Bis NHS Ester.

Procedure: A mixture of 60 mg of **11** (AD-155-5A, NGy5-OH) and 121 mg of DSC in 75 uL pyridine and 2 mL DMF is heated to 55°C overnight. The reaction mixture is washed with dry dichloromethane twice and dried to give the desired product **12**, NGy5-bis NHS ester.

### Example 11. Synthesis of NGy3-OMe.

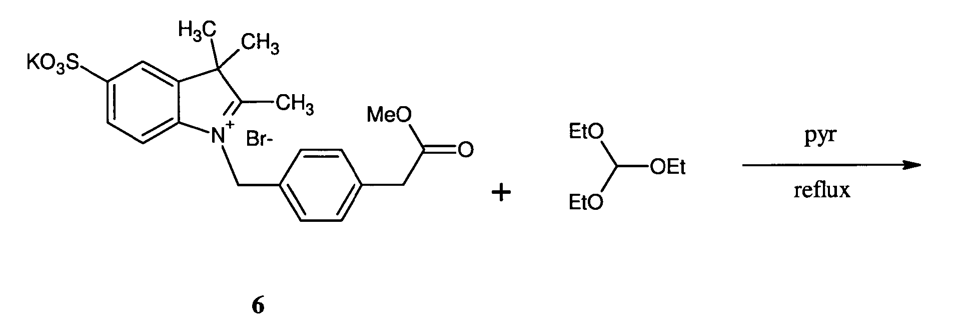

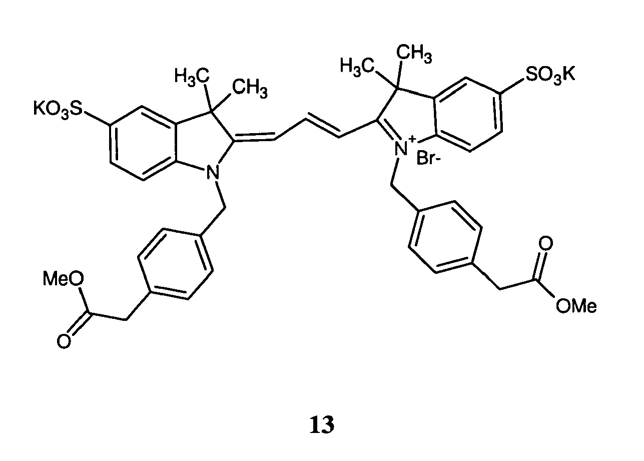

Procedure: A mixture of 521 mg of compound **6** and 832 ul of triethylorthoformate is heated to reflux in 2 ml pyridine to produce the desired product **13**, NGy3-OMe (AD-155-9).

### Example 12. Synthesis of NGy3-OH.

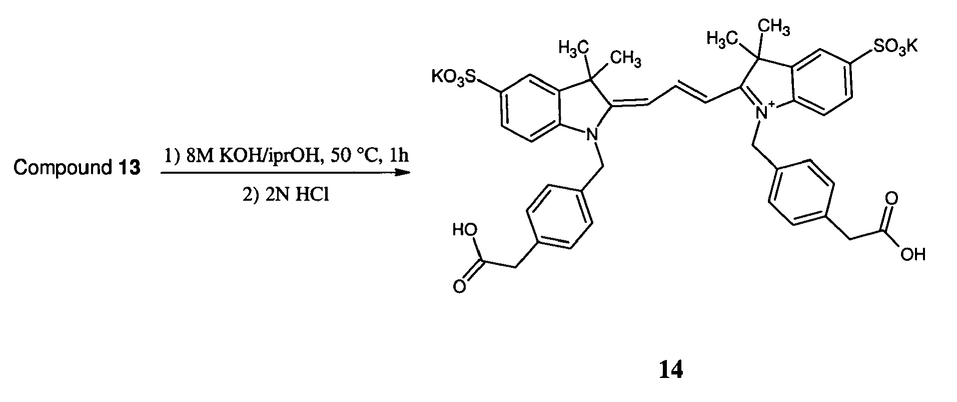

Procedure: A mixture of 140 mg of 13 (NGy3-OMe, AD-155-9) and 0.3 ml of 8M KOH/isopropanol in 2 mL of water is heated to 50°C for 1 hour. Additional KOH/isopropanol is then added if the hydrolysis has not gone to completion. HCl is then added to the mixture, which is then concentrated in vacuo to give the desired product **14**, NGy3-OH (AD-155-11C). Further purification may be desired.

### Example 13. Synthesis of NGy3-bis NHS ester.

Procedure: A mixture of 0.10g of 14 (NGy3-OH, AD-155-11) and 150 mg DSC in DMF/PY is heated to 55°C for 5 hours. The mixture is washed with dry dichloromethane and concentrated in vacuo to give the desired product 15, NGy3-Bis NHS ester.

### Example 14. Synthesis and Characterization of Dye Conjugates.

**Phycoerythrin conjugates**: NGy5, NGy7 and Gy5 dye stocks are resuspended, their absorbances determined and their concentrations calculated. Phycoerythrin (PE) aliquots (2 nmoles) are treated in 0.5 M sodium carbonate pH 9.0 for 1.5 hr at room temperature with: a) 1:15, 1:30, 1:50 or 1:80 fold molar excesses of NGy5; b) 1:15 or 1:30 fold molar excesses of NGy7; or c) 1:30 or 1:50 molar excesses of Gy5. After conjugation, each reaction mixture is loaded on a PD-10 column and eluted with PBS. Fractions containing protein are pooled together for each mixture. Absorbance and fluorescence spectra of the products are obtained and energy transfer efficiency assessed.

**Streptavidin conjugates**: The concentration of a dye solution is determined through absorbance readings of appropriate dye dilutions. Streptavidin is treated with a molar excess (e.g., 20 fold excess) of dye in sodium carbonate buffer (pH 9.0) for one hour. After conjugation, the mixture is purified on a PD-10 column using PBS as an eluent buffer, and 0.5 mL fractions are collected. Major fractions containing the streptavidin-dye conjugate are pooled together and absorbance and emission spectra are acquired.

### NGv5 Conjugates

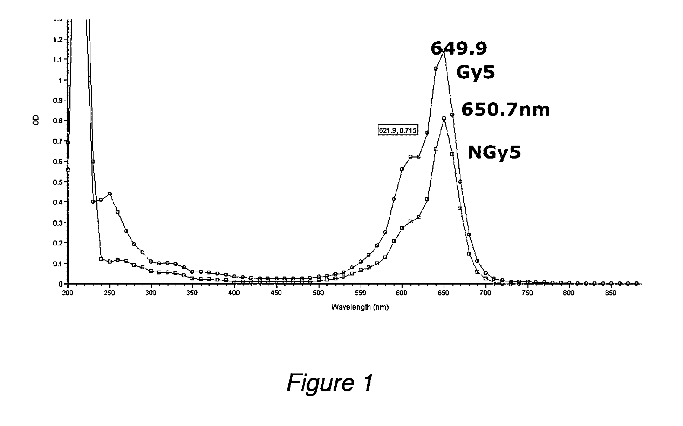

NGy5 shows very similar spectral characteristics to Gy5 (USPN 7,091,348, Guava Technologies) and Cy5. The absorption maximum of NGy5 is at 650.7 nm versus 649.9 nm for Gy5 (Figure 1). The dye shows very good solubility and no precipitation of conjugates after conjugating it to proteins was observed. NGy5 can be successfully conjugated to Phycoerythrin. The free unreacted NGy5 showed good separation from PE-NGy5 conjugates when purified on a PD-10 column, and the conjugate did not precipitate, indicating useful properties for derivatization of proteins and other biomolecules.

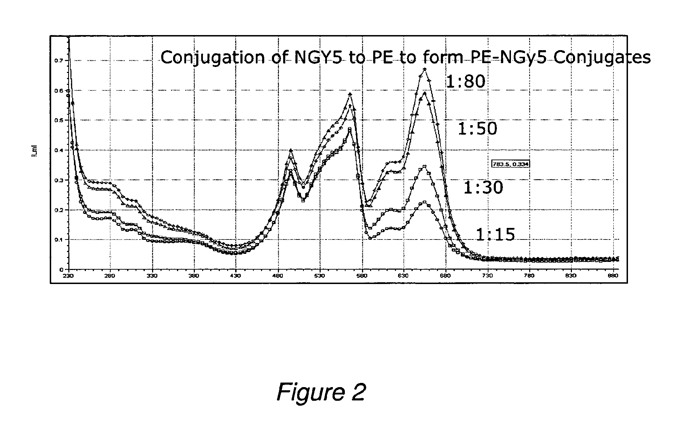

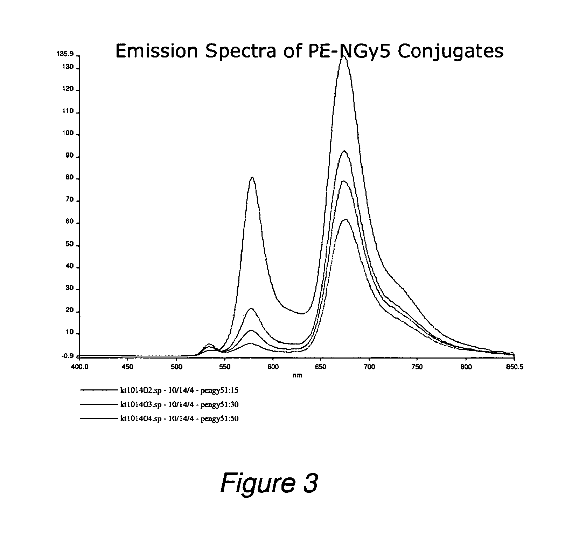

**Energy-transfer Conjugates**: PE demonstrates good conjugation and derivatization with NGy5 as evidenced by the increase at 646 nm in absorbance spectrum when coupled with different ratios of NGy5 (Figure 2). When the fluorescence spectrum of the same PE-NGy5 conjugates is examined, good energy transfer characteristics are observed, with approximately 90% energy transfer taking place (Figure 3). The resulting PE-NGy5 conjugates can be excited at 488 nm or 532 nm giving rise to an energy transfer conjugate that emits at -676 nm. Best transfer characteristics were observed at an input ratio of 1:50 or greater where -88% energy transfer was observed, and at a 1:80 ratio where ∼92% energy transfer is observed. While there is some quenching with conjugation of further NGy5, there is not a drastic reduction in fluorescence.

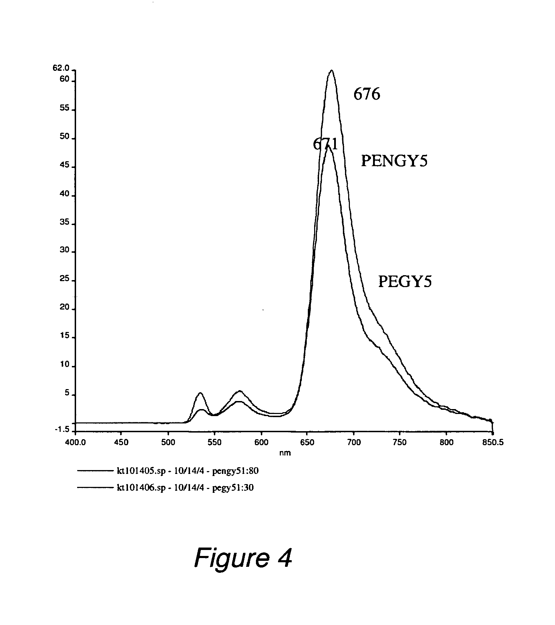

At similar dye/protein ratios, reduced fluorescence was observed in PE-Gy5 conjugates (Figure 4). The data suggests that NGy5 is a brighter dye than Gy5.

In addition to forming energy-transfer conjugates that can be used with a blue or green laser and emit in the red, NGy5 can also be used for direct conjugation to biomolecules such as proteins, antibodies, oligonucleotides and oligosaccharides, including conjugates that can be excited with a red laser.

### NGy7 Conjugates

The absorbance spectrum of NGy7 was compared to Cy7. NGy7 has an absorbance maximum of 752 nm, similar to the Cy7 absorbance maximum at 751.8 nm.

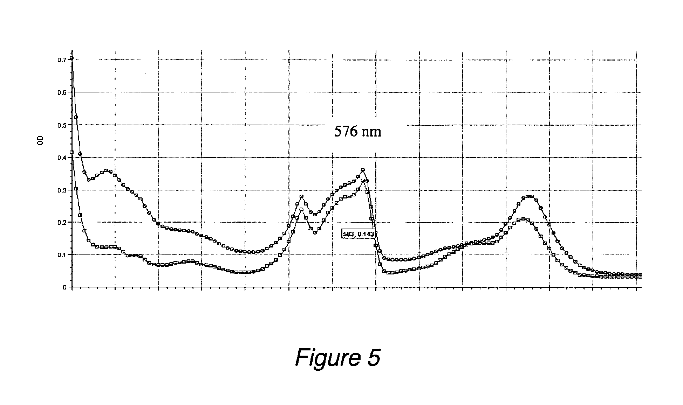

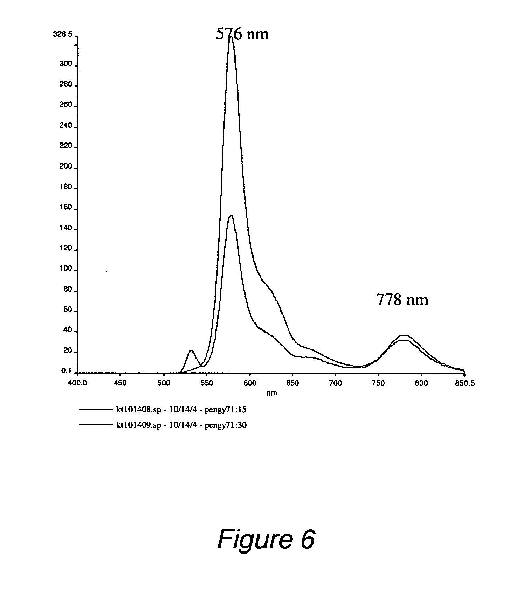

**Energy Transfer Conjugates:** NGy7 could be successfully conjugated to PE as evidenced by the absorbance spectrum. NGy7 shows good utility in conjugating to proteins in that it is soluble in buffered solution, its conjugates show good solubility and it can be separated from its protein conjugates very well on a PD-10 column. The absorbance spectrum of PE-NGy7 as shown in Figure 5 shows that increasing amounts of NGy7 can be successfully conjugated to PE. The fluorescence spectrum of the PE-NGy7 conjugate shows that that the complex can be excited at 532 nm and emits at 778 nm, indicating energy transfer is taking place. The spectrum shows that some of the PE energy is being transferred to NGy7 (Figure 6). Data should be acquired on a red sensitive fluorescence detector to truly estimate the fluorescence intensity at 778 nm and to determine the percent transfer. Further conjugation of NGy7 did not quench the NGy7 fluorescence.

### NGy3 Conjugates

NGy3 absorbance and fluorescene spectra were obtained and then compared to Cy3. NGy3 has an absorbance maximum of 556 nm and emits maximally at 572 nm. Its characteristics are very similar to Cy3.

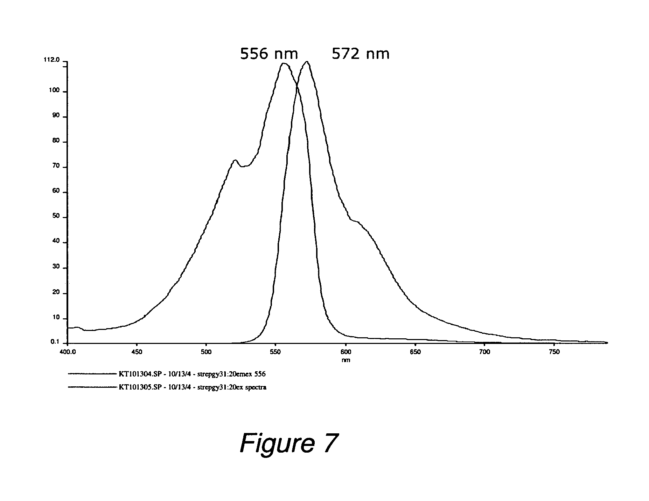

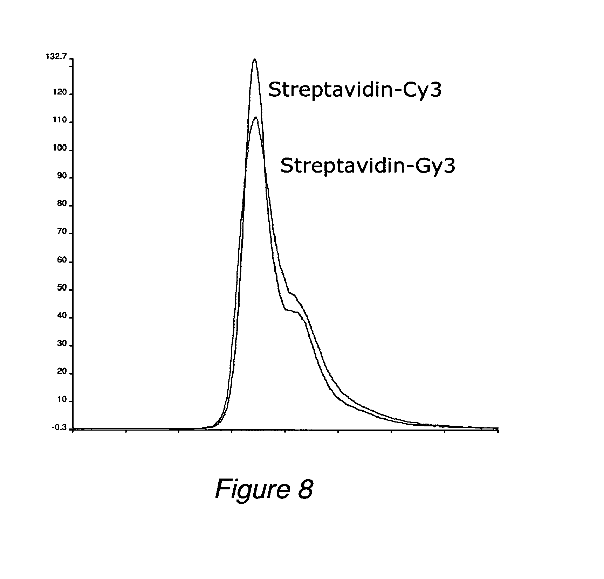

**Streptavidin-NGy3 Conjugates:** 5 nmoles of Streptavidin is treated with a 1:20 molar excess of NGy3 dye in sodium carbonate buffer (pH 9.0) for 1 hr. After conjugation the reaction mixture is loaded on a PD-10 column and the conjugated proteins eluted in phosphate-buffered saline (PBS). Fractions containing protein are pooled together. Absorbance (Figure 7) and fluorescence spectra of the streptavidin-NGy3 conjugate were obtained (Figure 8). The fluorescence spectrum clearly shows that the spectral characteristics of streptavidin-NGy3 are very similar to streptavidin-Cy3, and should be excitable with a green laser.

Although the invention has been described in some detail with reference to the preferred embodiments, those of skill in the art will realize, in light of the teachings herein, that certain changes and modifications can be made without departing from the spirit and scope of the invention. Accordingly, the invention is limited only by the claims.

## Claims

1. A conjugate comprising a compound of the formula: 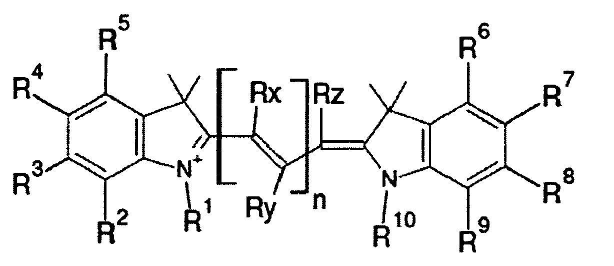 where:
n is zero, 1, 2 or 3;
R¹ and R¹⁰ are independently selected from the group consisting of optionally substituted alkyl, optionally substituted heteroalkyl, and optionally substituted alkylaryl, wherein at least one of R¹ and R¹⁰ is substituted alkylaryl comprising on the aryl component a substituted alkyl or heteroalkyl substituent comprising a carboxylic acid substituent;
R² to R⁹ are independently selected from the group consisting of H, - SO₃H, optionally substituted alkyl, or optionally substituted heteroalkyl, wherein any two adjacent members of R² to R⁹ taken together can form an optionally substituted 5-7 membered mono- or polyunsaturated fused ring optionally containing one or more ring heteroatoms;
Rz and each repeat of Rx and Ry are independently selected from the group consisting of H, alkyl, alkoxy, heteroalkyl, heteroalkyloxy, -CN, or wherein any two adjacent members of Rx, Ry, and Rz may be covalently joined to form an optionally substituted 4-7 membered mono- or poly-unsaturated ring optionally containing one or more ring heteroatoms;
or an isomer, ester, amide, acid halide, acid anhydride, and/or salt thereof;
wherein the compound is bound through a member of a coupling pair at any of R¹ to R¹⁰ to a second species selected from a probe, a linker, a target, a polymer, a biomolecule, a low affinity false target, one member of a binding pair, an optically active species and/or a substrate.

2. The conjugate of claim 1, wherein the second species is an optically active species with which the compound can exchange energy.

3. The conjugate of claim 1, wherein the second species is:
a) a biomolecule;
b) a synthetic dye; or
c) a semiconductor nanocrystal.

4. The conjugate of claim 1, wherein the conjugate comprises a substrate.

5. The conjugate of claim 4, wherein the substrate is
a) a bead; or
b) an irregularly shaped particle.

6. The conjugate of claim 1, wherein the conjugate comprises a polymer.

7. The conjugate of claim 6, wherein the polymer is
a) a dendrimer; or
b) a polyalkylene oxide.

8. The conjugate of claim 1, wherein the conjugate comprises a probe and, optionally, a low affinity false target.

9. The conjugate of claim 1, wherein the conjugate comprises one member of a binding pair, wherein preferably said one member of a binding pair is:
a) an antibody; or
b) a polynucleotide.

10. The conjugate of claim 1, wherein:
R¹ and R¹⁰ are each substituted benzyl or substituted phenethyl comprising on the aryl component a substituted lower alkyl or lower heteroalkyl substituent comprising a carboxylic acid substituent;
R⁴ and R⁷ are each sulfonic acid;
or an isomer, ester, amide, acid halide, acid anhydride, and/or salt thereof.

11. The conjugate of claim 1, wherein the conjugate is formed from a compound having a structure selected from the group consisting of: 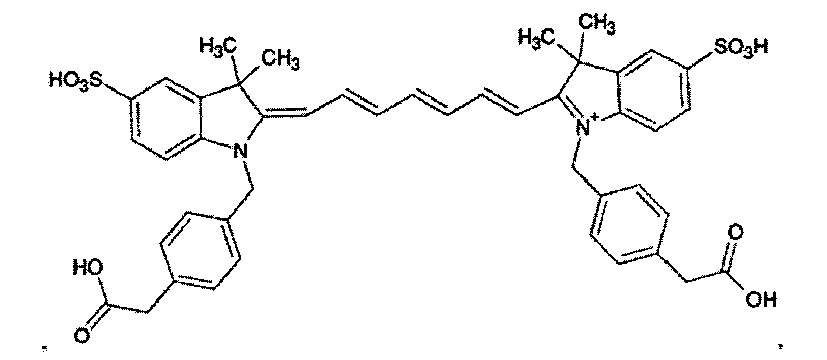 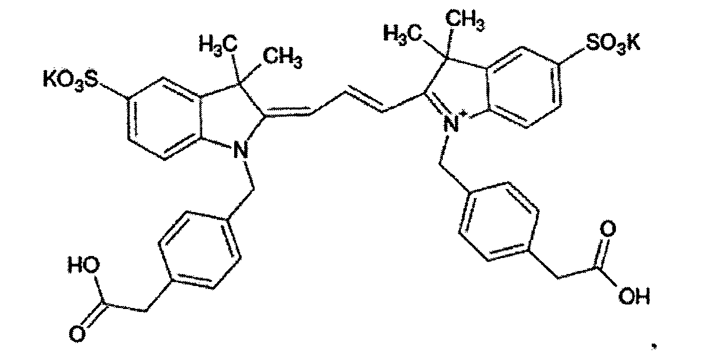 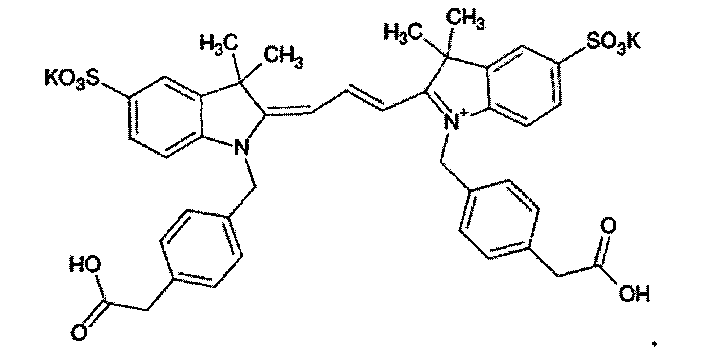 and an ester, amide, acid halide, acid anhydride, isomer, and/or salt of any thereof, wherein the compound as shown is conjugated through at least one of the carboxylic acid groups on the substituted alkylaryl to at least one of said second species.

12. A method of analyzing a sample for a target, comprising:
providing a sample suspected of containing a target;
contacting the sample with the conjugate of claim 1, wherein the conjugate comprises a probe, under conditions in which the probe can bind to the target, if present, to form a detection complex; contacting the sample or a fraction thereof suspected of comprising the detection complex with energy that can be absorbed by or transferred to the compound; and
determining if energy has been absorbed by or transferred to the complex.

13. The method of claim 12, wherein the conjugate further comprises a low affinity false target for the probe, and the target, if present, displaces the low affinity false target from the probe.

14. A detection complex formed by the method of claim 12.

15. The detection complex of claim 14, wherein the compound is in an excited state formed by contacting the complex with radiation that is absorbed by or transferred to the compound.
